# EUROPEAN PATENT SPECIFICATION

(11) **EP 0 591 289 B1**
(45) Date of publication and mention of the grant of the patent: **07.07.1999**
(21) Application number: 92912589.6
(22) Date of filing: 18.05.1992
(51) Int. Cl.: A61B 5/02, A61B 5/00, A61B 5/0484

(54) **HEMOGLOBINOMETERS AND THE LIKE FOR MEASURING THE METABOLIC CONDITION OF A SUBJECT**
HÄMOGLOBIN-MESSUNG ZUR BESTIMMUNG VON STOFFWECHSELGRÖSSEN EINER PERSON
HEMOGLOBINOMETRES ET ANALOGUES UTILISES POUR MESURER L'ETAT METABOLIQUE D'UN SUJET

(30) Priority: 16.05.1991 US 701127
(43) Date of publication of application: 13.04.1994
(73) Proprietor: NON-INVASIVE TECHNOLOGY, INC., Philadelphia, PA 19104 (US)
(72) Inventor: CHANCE, Britton, Marathon, Florida 33050 (US); ROBERTSON, Claudia S., Houston, Texas 77054 (US)
(74) Representative: Deans, Michael John Percy
(86) International application number: US9204153
(87) International publication number: WO9220273

(56) References cited:
- EP-A- 0 488 565
- WO-A-90/09003
- WO-A-92/13598
- GB-A- 2 228 314
- US-A- 2 790 438
- US-A- 4 013 321
- US-A- 4 029 085
- US-A- 4 224 948
- US-A- 4 321 930
- US-A- 4 469 107
- US-A- 4 700 708
- US-A- 4 738 267
- US-A- 4 869 224
- IEEE SPECTRUM, vol.20, no.3, 1983, NEW YORK (US) pages 52 - 57 'Watching the brain at work'
- NATURE, vol.324, no.6095, 27 November 1986 pages 361 - 364 GRINVALD A. ET AL. 'Functional architecture of cortex revealed by optical imaging of intrinsic signals'

## Description

This invention relates in one aspect to a method of deriving data signals useful for determining a physiological or pathophysiological property of a localized region of human tissue and to the provision of a spectrophotometer apparatus for examination of human tissue. In a second and alternative aspect of this invention, it relates to a method of deriving data signals useful in transcranial examination of brain activity and to a cognition spectrophotometer apparatus for transcranial examination of brain activity.

The increasing popularity of all forms of exercise over the last several decades has also led to an increased interest in the measurement of individual athletic performance. Generally heretofore, athletes were limited to obtaining heartbeat and blood pressure data while they were exercising. Although of some use, these data do not reflect peripheral circulatory capacity or the oxygenation state of specific muscle tissue. To measure oxygen delivery to the capillary bed of the muscles, an athlete needed to be tethered to an electrocardiogram apparatus and to have blood samples drawn while running on a treadmill. This was generally inconvenient.

In response to the evident need, the present applicants have developed a portable oximeter which relies upon the injection of light of a specific wavelength into the body of an individual and the detection of photons of light which have migrated through the tissue to a detector. The present invention in its various aspects has been developed from the applicants' portable oximeters and the relative technology.

Hamamatsu Photonics KK in GB-2 228 314A propose apparatus for examining an object of interest such as the head of a patient by transmission photometry. Hamamatsu employ a plurality of sequentially activated light sources arranged to direct into the object at respective incident sites via optical fibres. Light transmitted into and scattered within the object is detected at a plurality of output sites on the object. Signals from the sites are combined so as to determine optical absorption data at the incidence site then being addressed. Light from the output sites is combined a fibre optic arrangement and incident upon a common detector. In an alternative arrangement, it is suggested that the output sites could have respective detectors but in that case, the signals therefrom would simply be added together.

In accordance with a first aspect of the present invention, we provide a method of deriving data signals useful for determining a physiological or pathophysiological property of a localized region of human tissue of a subject, the method comprising the steps of: providing at least one light source, a first input port and a second input port for introducing electromagnetic radiation from said at least one light source to human tissue; and at least one light detector adapted operatively to detect, at a first detection port and a second detection port, radiation that has migrated in the tissue; the method being characterized in that said first input port and said second input port are arranged to introduce electromagnetic radiation of a selected wavelength from said at least one light source to said human tissue, in that said first input port and said first detection port are placed on the subject separated by a selected distance, at selected locations relative to the tissue to be examined, said distance and locations defining a first tissue region of interest; in that said second input port and said second detection port are placed on the subject separated by said selected distance, at selected locations relative to the tissue to be examined, said distance and locations defining a second tissue region of interest of similar volume to said first tissue region; in that said at least one light source and said at least one detector are operated, whereby said detector(s) detect at the respective detection ports radiation from said light source of said selected wavelength that has migrated through the skin and tissue in the first tissue region and the second tissue region; in that signals derived by said at least one detector in response to radiation detected at said first detection port and said second detection port are processed to create first and second processed data influenced by absorption or scattering by the skin and tissue through which the radiation has migrated; and in that the first processed data is correlated with the second processed data.

In a second and alternative aspect of the present invention, there is provided a method of deriving data signals useful in transcranial examination of brain activity of a subject, the method comprising the steps of: providing a light source and an input port adapted for introducing electromagnetic radiation of a selected wavelength from the light source to brain tissue, and a light detector adapted operatively to detect, at a detection port, the introduced radiation that has migrated in the tissue; placing said input and detector ports on the head of the subject; and operating said light source and said detector to detect radiation from said light source of said selected wavelength that has migrated in a tissue region of the brain from said input port to said detection port; processing signals, derived by said detector in response to detected radiation that has migrated in the brain, to create processed data; characterized in that said operation step is performed at the same time as a brain activity is being stimulated; and in that said processed data is correlated with said stimulated brain activity.

The invention provides, in a third alternative aspect thereof a cognition spectrophotometer apparatus for transcranial examination of brain activity by measuring changes in electromagnetic radiation scattered and absorbed in a migration path in the brain of a subject, the apparatus comprising: a light source constructed to produce electromagnetic radiation of a selected wavelength adapted to be introduced into the brain at an input port associated with said light source and adapted to be placed at a selected location upon the exterior of the head; a light detector adapted operatively to detect, at a detection port placed at a selected location upon the exterior of the head, radiation of said selected wavelength that has migrated in the brain; and processing means, connected to said light detector and being adapted to process signals, derived by said detector in response to detected radiation from said light source that has migrated in the brain, to create processed data; the apparatus being characterised in further comprising stimulation means adapted for causing stimulation of a brain activity simultaneously with operating said light source and detector; and evaluation means adapted to derive an evaluated characteristic of brain activity by correlating said processed data with said stimulation.

Further, according to a fourth and further alternative aspect of this invention, we provide a spectrophotometer apparatus for examination of human tissue of a subject by measuring changes in electromagnetic radiation scattered and absorbed in a migration path in tissue, the apparatus comprising: a first input port and a second input port for introducing electromagnetic radiation to human tissue, and at least one light source coupled optically with the input ports for providing such electromagnetic radiation; a first detection port and a second detection port, and at least one detector coupled with said detection ports for detecting electromagnetic radiation received thereat; and a processor, including electronic circuitry, coupled to said detector to receive signals therefrom; the apparatus being characterised in that: said first input port and said second input port are each adapted for introducing electromagnetic radiation of a selected wavelength to said human tissue from said at least one light source; in that said first input port and said first detection port are constructed to maintain a first relative distance that serves to define an examination volume of a first tissue region when said ports are mounted on human tissue; in that said second input port and said second detection port are constructed to maintain a second relative distance that serves to define an examination volume of a second tissue region when said ports are mounted on human tissue; said first and second relative distances being substantially the same and said first and second tissue regions having similar volumes; said at least one detector being adapted operatively to detect at said first detection port radiation from said at least one light source that has migrated through skin and tissue in the first tissue region from said first input port to said first detection port and, at said second detection port, radiation from said at least one light source that has migrated through skin and tissue in the second tissue region from said second input port to said second detection port; and in that the processor is adapted to process said received signals to derive first and second processed data, respectively corresponding to radiation detected at said first and said second detection ports that has been influenced by absorption or scattering by the skin and tissue through which the radiation has migrated and also to correlate said first data and said second data to provide an indication of a physiological or pathophysiological property of said tissue examined.

The invention in its third alternative aspect may be related to specific features of particular embodiments of apparatus in accordance with the invention which are described in detail hereinbelow with reference to individual figures of drawing as follows:

A cognition spectrophotometer apparatus for transcranial examination of brain activity by measuring changes in electromagnetic radiation scattered and absorbed in a migration path in the brain of a subject in accordance with the present invention comprises: a light source (200) constructed to produce electromagnetic radiation of a selected wavelength adapted to be introduced into the brain at an input port associated with said light source and adapted to be placed at a selected location upon the exterior of the head; a light detector (202) adapted operatively to detect, at a detection port placed at a selected location upon the exterior of the head, radiation of said selected wavelength that has migrated in the brain; and processing means (210, 212, 214), connected to said light detector and being adapted to process signals, derived by said detector in response to detected radiation from said light source that has migrated in the brain, to create processed data; the apparatus being characterized in further comprising stimulation means (218) adapted for causing stimulation of a brain activity simultaneously with operating said light source and detector; and evaluation means (218) adapted to derive an evaluated characteristic of brain activity by correlating said processed data with said stimulation.

Similarly, the invention in its fourth alternative aspect may be related to specific features of individual embodiments of apparatus constructed in accordance with the present invention which are described in more detail hereinbelow somewhat as follows:

A spectrophotometer apparatus for examination of human tissue of a subject by measuring changes in electromagnetic radiation scattered and absorbed in a migration path in tissue in accordance with the present invention comprises: a first input port (271) and a second input port (272) for introducing electromagnetic radiation to human tissue, and at least one light source (228) coupled optically with the input ports for providing such electromagnetic radiation; a first detection port (271) and a second detection port (272), and at least one detector (230) coupled with said detection ports for detecting electromagnetic radiation received thereat; and a processor (234, 236, 238, 240, 274), including electronic circuitry, coupled to said detector to receive signals therefrom. The apparatus is characterized in that: said first input port (271) and said second inpout port (272) are each adapted for introducing electromagnetic radiation of a selected wavelength to said human tissue from said at least one light source (228); in that said first input port and said first detection port (271) are constructed to maintain a first relative distance that serves to define an examination volume of a first tissue region when said ports are mounted on human tissue; in that said second input port and said second detection port (272) are constructed to maintain a second relative distance that serves to define an examination volume of a second tissue region when said ports are mounted on human tissue; said first and second relative distances being substantially the same and said first and second tissue regions having similar volumes; said at least one detector (230) being adapted operatively to detect at said first detection port radiation from said at least one light source (228) that has migrated through skin and tissue in the first tissue region from said first input port to said first detection port and, at said second detection port, radiation from said at least one light source that has migrated through skin and tissue in the second tissue region from said second input port to said second detection port; and in that the processor (274) is adapted to process said received signal to derive first and second processed data, respectively corresponding to radiation detected at said first and second detection ports that has been influenced by absorption or scattering by the skin and tissue through which the radiation has migrated, and also to correlate said first data and said second data (234, 242, 244) to provide an indication of a physiological or pathophysiological property of said tissue examined.

As we shall explain below, the present invention enables a study of the linkage between cerebral activity and oxygen delivery and oxidative metabolism in the brain tissue. Heretofore, during a brain activity, blood flow could be studied using PET or NMR. Faster electrical and magnetic responses could be measured using EEG and MEG. Such techniques might eventually be able to provide examination and screening for neuronal deterioration and/or deterioration of brain function, but they are relatively expensive and not suitable for emergency treatment situations wherein diagnostic equipment should be taken to a patient. As we shall explain below, optical techniques provide a suitable, cost effective alternative for examination and screening of a tissue of an organ. In embodiments of apparatus in accordance with the present invention in which there are two input ports and two detection ports, the radiation may be introduced simultaneously at the two input ports or sequentially at the first input port and detected at the first detection port, and subsequently introduced at the second input port and detected at the second detection port.

In embodiments having two input ports and two detection ports and adapted for cognition spectrophotometry or for examination of the brain, the first input and the first output port are suitably located on one parietal bone (or temporal bone) separated by a predetermined distance, and the second input port and the second output port are similarly located on the other parietal bone (or temporal bone). The processed data may be correlated by generating a differential signal or by forming a Fourier transformation.

The stimulation means may be adapted to cause visual stimulation, acoustic stimulation, or sensorimotor stimulation.

The evaluation means can be adapted to examine pathophysiological properties of the brain tissue or cognitive function of a selective region of the brain based on correlation between the processed data the caused stimulation of the brain activity.

The system's first and second light sources are suitably tungsten lamps or light emitting diodes. The first or second detectors are suitably silicon diodes or light-to-frequency converters, each with an interference filter adapted to detect the radiation of the selected wavelength.

The processing means suitably comprise a differential counter adapted to register differential signals received from the light-to-frequency converters. Clocking means adapted to route signals of the detected radiation from the light-to-frequency convertors to the differential counter, a frequency-to-voltage converter adapted to convert signals from the differential counter and/or a fast Fourier transformer adapted to process differential signals from the frequency-to-voltage converter.

The evaluation means suitably comprises a storage oscilloscope adapted to analyze the Fourier transformed differential signal of the fast Fourier transformer, and/or computational means adapted to analyze the differential signal.

Where the methods of the present invention are employed to derive data signals useful to determining a physiological or pathophysiological property of a localized region of human tissue, the tissue tested can be the brain, a breast, or a limb. In that case, the first input port and the first detector are located in appropriate positions associated with that side of the brain, that breast or that limb whilst the second input port and the second detector are similarly located on the other side of the brain, the other breast or the corresponding limb.

The invention is hereinafter more particularly described by way of example only with reference to the accompanying drawings, in which:-
FIG. 1 is a block diagram of a low frequency cognition spectrophotometer constructed in accordance with the present invention;
FIG. 2 is a plan view of a preferred embodiment of sensor;
FIG. 3a is a plan view of the oximeter sensor of FIG. 2;
FIG. 3b is a longitudinal side view of the oximeter sensor of FIG. 3a;
FIG. 3c is a longitudinal cross-sectional view taken along the line 6-6 in FIG 3a;
FIG. 4 is a transverse cross-sectional view of an oximeter sensor in place upon the flesh of a wearer;
FIGS 5a, 5b and 5c are plan views of other preferred embodiments of the oximeter sensor;
FIG. 6a is a plan view of an implantable oximeter sensor;
FIG. 6b is a longitudinal side view of the sensor of FIG. 6a;
FIG. 6c is a cross-sectional view taken on line 9c-9c of FIG. 6a;
FIG. 7 is a block diagram of an analog version of a control system for use with the oximeter sensors of the foregoing figures;
FIG. 7a is a schematic representation of the oximeter control system shown in FIG. 7;
FIG. 7b is a section of the schematic representation shown in FIG. 7a;
FIG. 8 is a block diagram of a digital version of control circuit for an oximeter sensor as illustrated in the foregoing figures;
FIG. 9 is a software flow diagram of the software used with the circuitry of FIG. 8;
FIG. 10 is a front view of a helmet employing sensors as described hereinbefore;
FIG. 11 illustrates the response of the Fourier transform oscilloscope of the cognition spectrometer shown in FIG. 1 to 300 µV peak-to-peak square wave (top trace) and 225 µV sine wave (lower trace);
FIG. 12 illustrates the response of the DC amplifier to a square wave;
FIGS. 13a and 13b show frequency analyses of optical signals of subject U obtained by the cognition spectrophotometer of FIG. 1;
FIGS. 14a and 14b show similar analyses for subject B;
FIGS. 15a and 15b show frequency analyses of optical signals of subject U at rest obtained by the cognition spectrophotometer of FIG. 1;
FIG. 16 shows a histogram display of the distribution of frequencies obtained by the cognition spectrophotometer of FIG. 1;
FIG. 16a shows a histogram display of the distribution of energy (area) in the peaks obtained by the cognition spectrophotometer of FIG. 1;
FIG. 17 is a block diagram of a fast lateralization detector of the cognition spectrophotometer of FIG. 1;
FIG. 18 is a block diagram of a low frequency lateralization detector of the cognition spectrophotometer of FIG. 1; and
FIG. 19 is a diagrammatic view of an arrangement for use with the arrangements of FIGS. 17 or 18.

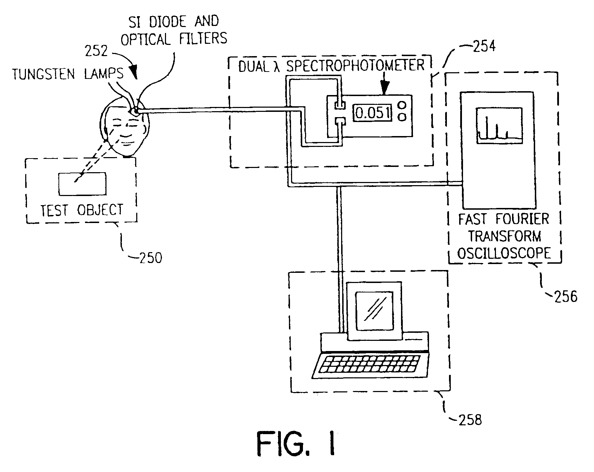

A preferred embodiment cognition spectrophotometer system, constructed in accordance with the present invention is shown diagrammatically in Figure 1. The cognition spectrophotometer examines the relationship between brain activity, oxygen delivery, oxidative metabolism, and blood flow by employing photon migration from a source placed on the surface of the head through the skin, skull and underlying brain tissues to a detector also located on the surface of the head. Light is absorbed and scattered along the migration path.

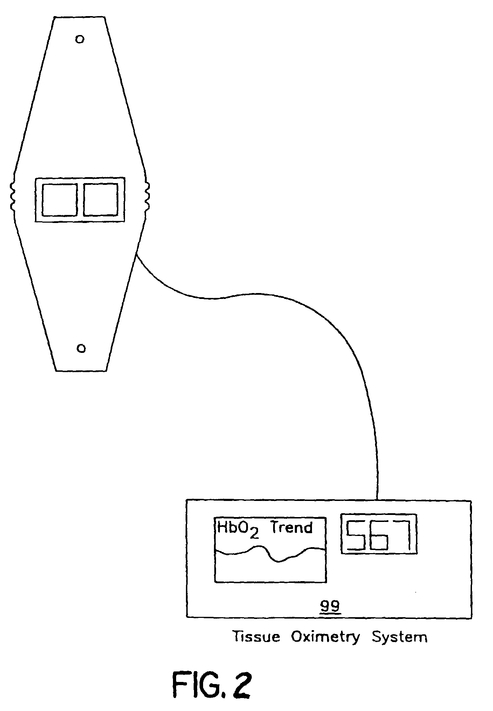

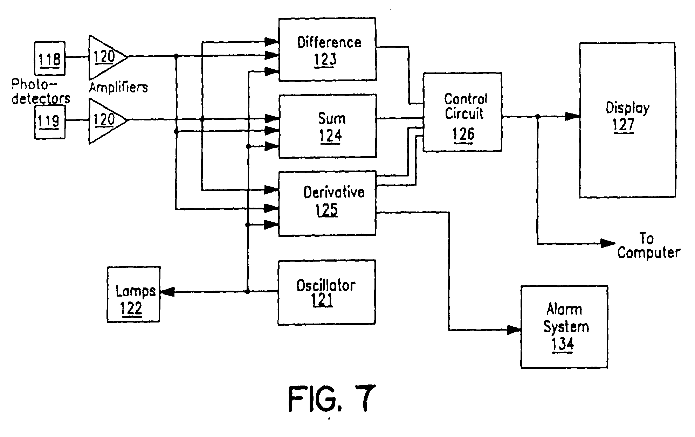

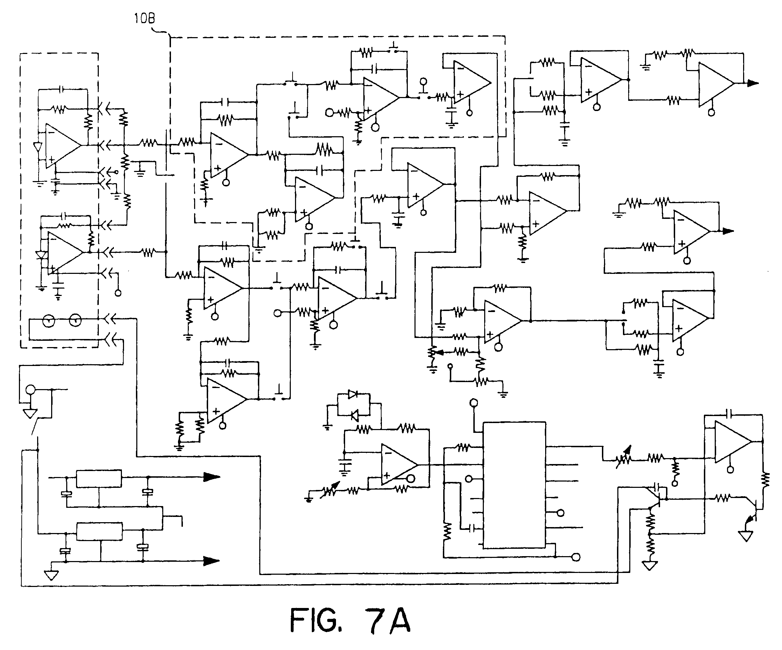

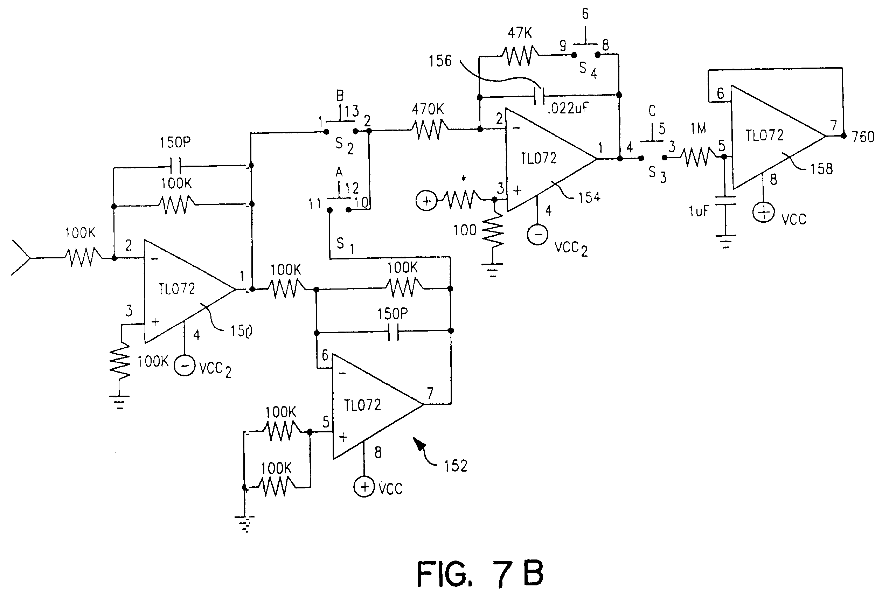

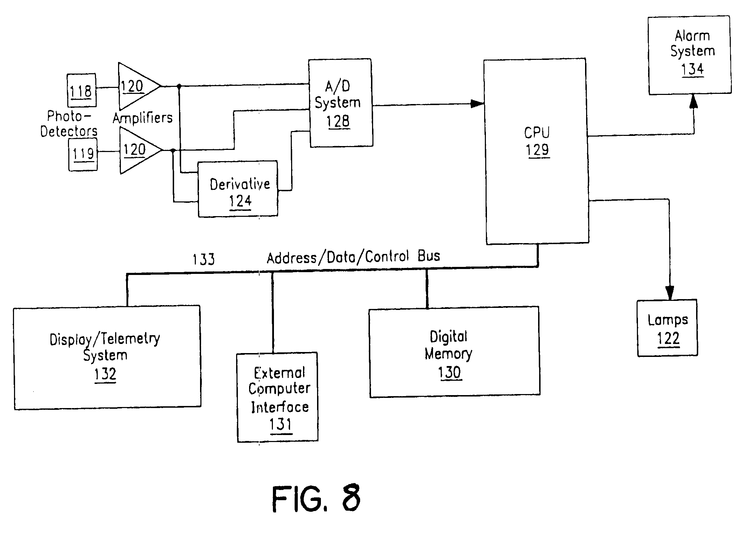

Referring to Figure 1, the system comprises the following five modules: a stimulation module 250, a sensor module 252, a control circuit module 254, an analyzer/display module 256, and a computer/printer module 258. Stimulation module 250 is used to stimulate a specific brain function through a visual, acoustic, sensorimotor, or other stimulation. Sensor module 252 is connected to control circuit module 254; preferred embodiments are shown in Figures 2 to 10, 17 and 18. Figures 7 and 8 diagrammatically show the sensor, control circuit, display and computer/printer modules. The display module of Figure 1 is a fast Fourier transform oscilloscope. The operation of sensor module 252 is governed by control circuit module 254 which transform oscilloscope. The operation of sensor module 252 is governed by control circuit module 254 which controls the radiation emission and detection, collects the data, and sends the data to analyzer/display module 256 and to computer/printer module 258. The operation of the whole cognition system is controlled by a computer of computer/printer module 258.

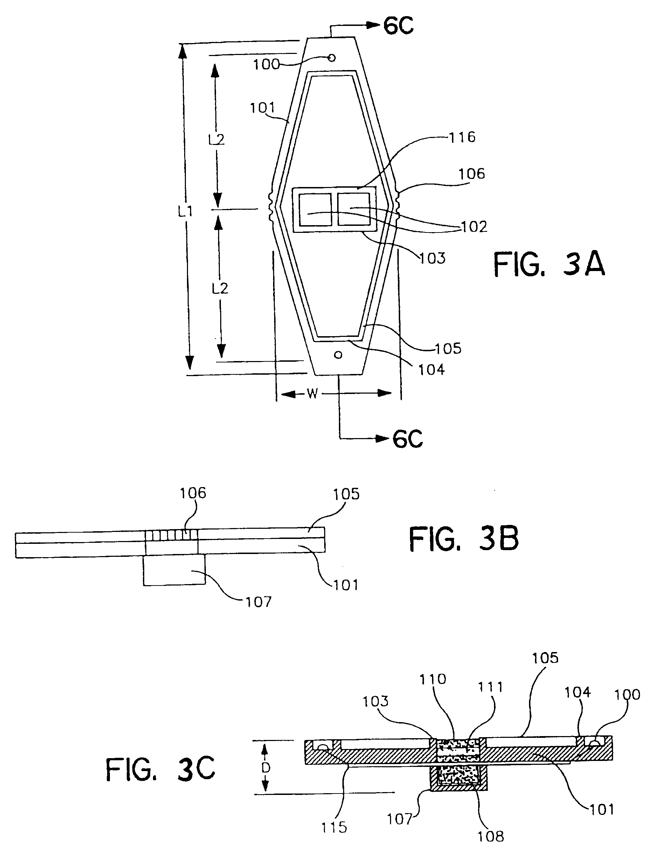

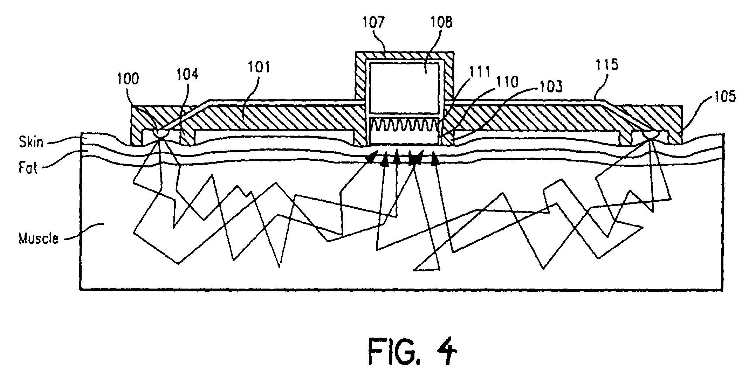

In Figures 2 and 3a-3c, a rubber-backing member 101, provides support for two lamps 100 spaced equidistant from two detectors 102 also mounted on backing member 106. The backing member is formed of an opaque, e.g., black, silicone rubber of suitable durometer to enable it to conform to the curvature of the subject part of the human body to which it is applied. For this embodiment, which may be as long (L₁) as e.g., 12, especially 8 centimeters, flexure configurations 106 are provided. Light barrier members 103, 104 serve to depress the subcutaneous fat layer and thereby reduce light interference directly between the light sources 100, located at input ports, and the detectors 102, located at detection ports, see description below regarding Figure 4. Behind the detectors 102 of Figure 3a, as shown in Figure 3c, housing 107, defined by the rubber wall, contains the supporting circuitry for these lamps and detectors. As shown in Figure 3c, narrow band optical filter 110, located at the detection port, lies over photodetector 111, which lies over circuitry 108. Depth D is typically 2 cm. Wiring harness 115 carries power to the lamp.

On the rubber supporting member 101 there are a number of integral raised members 103, 104, 105 and 106. Raised rib 105, which extends about the perimeter, both prevents external light from interfering with the reading and serves to support comfortably the backing member 101 on the subject. Rib 104 extending laterally, adjacent the lamp, and disposed across the line projected between the lamp 100 and the detectors 102, serves as a second light barrier to prevent interfering light transmission between light source 101 and detectors 102. Rib 103 closely surrounds the detectors, and serves as a primary eliminator of environmental light interference, and also serves to absorb light migrating along subcutaneous fat and other subsurface interposed layers, etc. All of these ribs are on the order of 1/2 centimeter high and 1/2 centimeter thick. Their outside flesh-engaging edges are rounded for comfort to the wearer. The supporting member 101 and its associated ribs are manufactured in one piece of molded rubber. A suitable mold is provided and black silicone rubber is poured into the mold, cured and dried, leaving the subsequent rubber backing 101 with integral ribs and structures. Suitable mounting sites are provided in the backing into which the detectors 102 and the lamp 100 are mounted during final manufacturing. The backing member for the oximeter sensor of Figures 3a-3c has width, W, length, L1, and depth D, which may be varied depending upon the application. L2 represents the spacing between light source 100 and the center of detectors 102. Sensors with-dimension (L₂) from one centimeter to four or five centimeters with corresponding changes in L1 and W are appropriate. L2 of four or five centimetres is useful for deeper tissue penetration for the brain.

Adjustability of the device of Figure 5c enables L₂ adjustment. It will also be realized that monitoring may be achieved through wound dressings, bandages, etc.

The light sources 100 are suitably lamps having tungsten filaments, and are broad band light sources which eliminate the problem of matching the light sources to the detector filters.

Each detector is comprised of interference filter 110 which blocks out all light except for that which is desired, each of two detectors having a separate wavelength of interest. At this time 760 nm and 850 nm are preferred, although one can envision that changing, depending upon the application. Beneath the filter is a photosensitive detector which picks up the light and transduces it to an electrical signal which is amplified in the circuit 108 and later transmitted to the control circuitry represented in either Figure 7 or 8.

In the presently preferred embodiment, the interference filter is manufactured by Omega, Inc., and the photodiode beneath it is Part No. F1227-66BR, available from Hamamatsu, having a large sensitive area for favorable signal to noise ratio and an NIR wavelength sensitivity. The sensitive area is approximately 6 millimeters squared.

In the present embodiment the filter and detector are epoxied together around and an electronic shield 115 surrounds the diode/filter pair 110 and 111. This surrounding electronic shield eliminates or reduces extraneous electronic interference. It is presently preferred to form this shield of copper in the form of a windowed box which surrounds the detector filter pair.

Once the two separate filter diode pairs are constructed, they are soldered together and then mounted directly to the circuit board 108. Connected also to circuit board 108 is an ultra low noise operational amplifier with high gain, which converts the current signal from the diodes to a voltage applicable to the control circuitry of Figures 7 or 8. The circuit board 108 can be connected via either telemetry or cabling to the oximetry system 99 of Figure 2, which contains the circuitry shown in Figures 7 or 8. The circuit board 108 can be connected via either telemetry or cabling to the oximetry system 99 of Figure 2, which contains the circuitry shown in Figures 7 or 8. Power supply for the amplifier of 108 is supplied by the oximetry system 99 where a cable connection is employed. In other embodiments, a battery is provided for operating the oximeter sensor along with the telemetry system, to be described below in connection with an implantable embodiment.

Referring now to Figure 4, the preferred embodiment of Figures 3a-3c is shown diagrammatically as it is placed upon the skin of a subject. The edges of the upstanding rib-form barrier members serve to concentrate pressure upon the skin, depressing the skin layer and the underlying fat layer. The barriers 103 and 104 serve to prevent light from migrating directly between the source 100 and the detectors 102 and because the barriers are placed with pressure upon the surface of the skin, they serve to reduce the area of the fat through which light can pass directly. If one were to imagine the situation without a barrier, one would see light passing almost directly between the source and the photodiodes, the fat layer serving, effectively as a light guide. The absorbing ribs reduce this noise effect. Light which is emitted by the sources 100 enters the skin directly beneath the source, passes through the fat to the underlying tissue, migrates through the tissue, is absorbed, scattered. and eventually is received by the photodiode. The migration path could be described as a banana-shaped path which is due to the photon migration between the source and the detector. "Banana-shaped" is a mean representation of the photon path, whereas the actual migration path constitutes many scattering changes of direction of the photons as they course between the light source, located at the input port, and the photodiode, located at the detection port.

Figures 5a-5c show alternate preferred embodiments of the oximetry sensor.

The embodiment of Figure 5a is shown here as a comparison to Figure 3a-3c, wherein the overall length L1 and the overall width W depends upon the application and L2 as in Figures 3a-3c can vary from one centimetre or less to five centimetres or more.

The overall length L1 is determined chiefly as a result of the source 100 to detector 102 spacings L2. The spacing determines the depth of penetration of the light which is scattered and migrated through the tissue. The farther the source is from the detector, the deeper the mean penetration. So for shallow penetrations, one would envision a short L2 and thereby L1. The penetration desired depends upon the tissue of interest.

The width of the sensor is chiefly dependent upon the size of the detectors 102. In the configuration of the presently preferred embodiment wherein each detector has a photosensitive area of approximately 6 millimeters squared, the width is dependent almost entirely upon those two dimensions. As the photodetectors reduce in dimension width W decreases.

The larger photodetector units provide better signal to noise ratio and thereby enable more accurate representation of the oxygenation state of the tissue. As improvements in technology occur and better photodetectors and initial amplification circuitry are developed, the detector size will decrease, with consequent decrease in W.

As with Figure 3a-3c, the supporting member 101 of Figure 5a carries numerous rib-form barriers. In this case barriers 103, 104 and 105 serve both support and light reduction functions. Perimeter barrier 105 in this case completely surrounds the light source and detector grouping. Between the light source and barrier 103, is barrier 104 on opposite sides of the detectors. Barrier 104, as previously mentioned, serves to reduce light as it travels between source and detector in the subcutaneous layer.

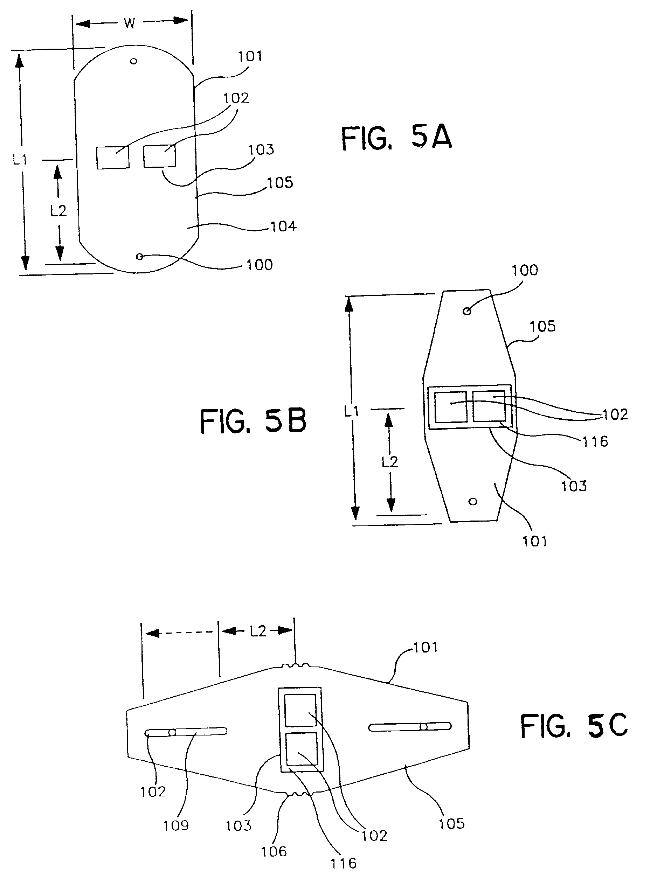

The embodiment of Figure 5b represents an alternate to that of Figure 5a wherein the dimensions of Figure 3a are significantly reduced to achieve a smaller probe. In addition to the backing member 101 being reduced in size; in Figure 5b, barrier 104 has been eliminated and barrier 103 serves as the primary and only eliminator of both external light and interference between source 100 and detector 102. The typical dimensions for L2 of Figure 5b would be 3 centimetres or less, L1 being 6 centimetres maximum or less. In comparison, the minimum size for the embodiment of Figures 5a and 3a-3c of L2 would be 3 centimetres or greater.

The embodiment pictured in Figure 5b is suitable to be used for example in neonate applications where the desired tissue volume is extremely small and one needs a small probe.

The smaller sensor sizes improve the flexibility of the device to correspond to smaller regions of interest.

Referring to Figure 5c, a similar embodiment to that of Figure 3a-3c is shown, but having a light source track 109 to enable variable spacing between the light source 100 and detector. Barrier 103 has been omitted in favor of allowing for user settable variations of L2. L2 may be varied between for example 2 centimeters to say 5 centimeters depending upon the application.

For this adjustability, a slide mechanism is employed in manner to keep L2 equal on both sides, in dependent motion such that as the spacing of one varies, the spacing of the other will also change.

The embodiments of Figures 2-5 share the desirable features of a parallel pair of detectors 102, side-by-side extending across the line between the light source. By simultaneous flashing of both lamps each detector receives photons at its wavelength from both lamps, simultaneously.

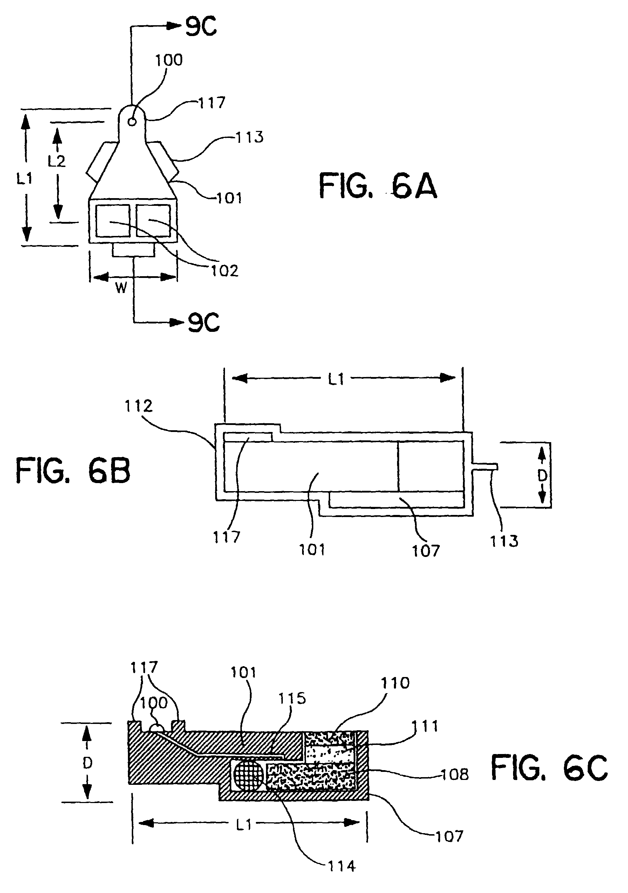

Figure 6 shows another preferred embodiment of the tissue oximeter sensor, in the form of an implantable probe. To further reduce size, one of the light sources 100 is omitted. As in Figure 5b, light barrier 104 is omitted. The lone barrier in this case 117 serves to reduce direct light interference.

As previously mentioned, backing member 101 holds in fixed relation the light source 100 and the detectors 102. The length L1 is solely dependent upon a single L2 between the single source and the dual detectors. The spacing depends chiefly upon the region which is being studied. As previously mentioned, from 1/2 centimetre or 1 centimetre to 5 centimetres may be appropriate, depending upon the application.

For application, the physician makes an incision in the skin and slips the oximeter sensor underneath the skin and cutaneous fat layer. There are suture points 113, e.g., biocompatible webbing, surrounding the backing member 101. A coating over the entire sensor is comprised of a biocompatible base material 112, which protects the circuitry from the human system, and protects the human from the invasive nature of the circuitry.

The thickness of the device is of the order of 1 to 2 centimeters maximum. That depth dimension will diminish as technology changes. In Figure 6c the supporting circuitry is shown. As previously described, the filter/photodiode pair 110, 111 is disposed above the circuit 108. In addition to receiving and amplifying the signal, the circuit shown here is responsible for telemetric communication of the signal to a receiver outside of the body. A battery 114 powers that circuitry.

By employing a radio signal to transmit the information from within the body to a receiver outside the body there is no need for wires and the like puncturing the skin.

Referring to Figure 7, one embodiment of the circuitry for driving the device is shown. (Schematic diagram of the embodiment of Figure 7 is shown in Figure 7a). This is an analog circuit wherein the signal from photodetectors 118 and 119 is amplified by amplifiers 120 and sent to three manipulative circuits that take the difference, the sum and the derivative of the signal. The difference 123, as described above, simply subtracts 760 nm minus 850 nm to obtain a signal representing deoxygenation.

The sum circuit 124 takes a weighted sum of the 760 nm and 850 nm signals, weighting being chosen appropriate to the fact that the signal variation due to oxygenation or deoxygenation is greater for 760 nm than it is for 850 nm. Because these contrabestic wavelengths tend to cancel the signal due to the difference in oxygenation, the sum shows independent of the difference and is taken as representative of the blood volume changes in the tissue.

The derivative circuit 125 takes the simple derivative to show the rate of change of both of the signals. This is useful, as described above, to trigger alarm circuitry based upon established standards, wherein the higher the rate of the change, and the more sustained that rate of change, the more potentially dangerous the rate of change. This is useful for example in monitoring aviators for possible black-out conditions and for apnoea.

The outputs of these units 123, 124 and 125 are applied to the control circuit which controls where the signals are directed and how they are displayed and/or sent to a computer. The control circuit may be simply embodied as a switch to switch the output to an LCD display, for example. The analog signal from control circuit can be digitized in the display unit 127 and displayed as a digital number. Additionally it can be digitized and sent to a computer or sent in analog form to a computer for digitization.

The oscillator 121 is an independent source for determining the frequency of lamp flashing. Lamps flash at frequency of 1/2 Hz or 2 flashes per second or greater. This frequency may be independent of heart rate.

The lamp rate is tied to the control circuit. The oscillator establishes the timing for the sum and difference circuits because the sum, difference and derivative circuits need to be synchronous. In operation, the lamp flashes, the signal is picked up by the photodetectors and while the lamps are on, the difference, sum and derivative are calculated and are thereby stored in the appropriate memories, and via the control circuit can be directed to the display and to the computer.

The derivative system is the basis of the alarm system. Output from the derivative is compared to a standard within the alarm circuitry, which then determines if there is, for example, a normal rate of change, represented say by a green light, a cautionary rate of change, which may be represented by a yellow light, and a fairly rapid and/or sustained rate of change, which would be for example shown by a red light, an alarm or a buzzer or the like, which would alarm both the wearer or act remotely for example to warn the parents of a neonate in the case of SIDS (Sudden Infant Death Syndrome).

The embodiment shown in Figure 7 enables correction for the dark current/noise that comprises background light, DC offset of the operational amplifiers, photodiode dark current, temperature effects on the outputs of individual components and variations due to changing environment. The dark current/noise correction is explained on the circuit of Figure 7 which is a section of the analog circuit, shown in Figure 7. The oximeter (Figure 7) performs data acquisition in four steps which are synchronized by an internal oscillator. In the first step, the lamps are off. The output is directed to an integrator 154 and integration capacitor 156 is charged to the dark level voltage. In the second step, the lamps are turned on. The preamplifier output that corresponds to the intensity of the detected light is directed to integrator 154 in a way to charge capacitor 156 with current of polarity opposite to the polarity of the charging current in the first step. This is achieved using appropriate ON/OFF combination of switches S1 and S2. The voltage of capacitor 156 is charging to a value which, at the end of this step, represents the total signal minus the dark level noise signal. In the third step, both switches S1 and S2 are turned OFF to disconnect both the positive unity gain and the negative unity gain operational amplifiers (150 and 152). Then, the output of integrator A is moved via switch S3 to a hold circuit 158 which also functions as a low pass filter. This output is the detected signal at one wavelength corrected for the background noise. In the fourth step, the switches S1, S2 and S3 are open and switch S4 is closed in order to discharge capacitor 156 through a 47K resistor. At this point, the circuit of integrator 154 is reset to zero and ready for the first step.

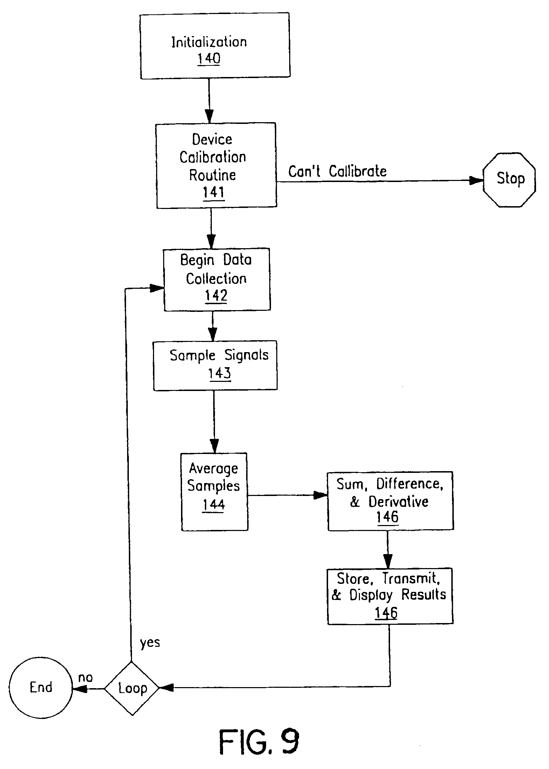

A digital version of the circuitry is depicted in Figure 8. The identical photodetectors 118 and 119 and similar amplifiers output signal to an analog to digital conversion system 128 and to a derivative circuit 124. The derivative circuit outputs signal to the analog digital converter, in this case for evaluation by the central processing unit, CPU, or microprocessor 129. Software, shown in Figure 9, controls the system of data collection and lamp frequency 122 as well as the storing of data, interfacing with external computers and displaying or telemetrically communicating this information. The heart of this circuit is the central processing unit driven by software which collects data, stores it, displays it and sounds alarm if necessary.

Figure 9 shows the software. Initialization of the system 140 takes place whereby the analog and digital system is set up and configured properly. The digital memory, communication and telemetry are configured as in Figure 8. Secondly the device calibration takes place such that the gain of the amplifiers is set electronically by software. The gain of the amplifiers is set to an acceptable range so that digitization can take place accurately, as well as other small internal routines to determine whether the derivative is working properly or not. In the case that the calibration cannot take place, the program will stop and alarm the user. The alarm 134 represents "not working properly, please reset", etc. After calibration is completed successfully, data collection is begun. Data collection is taken in a loop format starting with 142. It starts with turning the lamp on, and sampling the signal, 143. Approximately 500 points of data are taken in rapid succession over approximately 1/2 second sampling interval or less. That data is accumulated, then the lamp is turned off after a delay period, which is set by the user and by the software. The samples are collected and then averaged at 144. This average is then used at 145 to calculate the sum, difference and derivative. In this case the calculated derivative serves as a redundant comparison with the analog derivative calculated in 125 of Figure 8. In addition to the averaging of 760 and 850 nm, the derivative signal is also averaged and sampled in the same way, for example with 500 points. By this means a calculated derivative as well as a sample derivative are obtained which are compared to provide a much more repeatable and reliable result for an alarm.

The data after it has been manipulated in 145 will be stored, appropriately transmitted and/or displayed. In addition the alarm is set off if necessary at this point. Then finally an independent timer or delay would be introduced. The processor is delayed for a set period to obtain desired lampflash/data collections frequency.

The sequence is thus: lamp on, collect sample data, lamp off, collect noise data, average sample, calculate sum, difference and derivative, then transmit, display etc., wait if necessary, and then turn on the lamp again and repeat the whole procedure.

Referring to Figure 8, dark current/noise correction in this analog system is accomplished by sequencing the data collection using the microcontroller. The data is collected in two sequencing steps. One step has light on and the other step has light off. The data collected with the light off represents the dark current. The data collected in the first and in the second cycle are digitally subtracted in order to obtain intensity data corrected for the dark current noise.

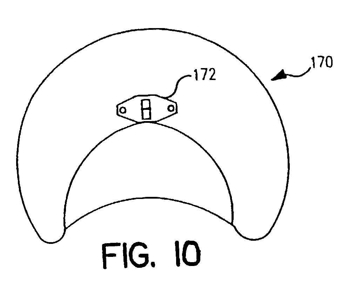

Referring now to Figure 10, a helmet 170 is shown having a tissue oximeter 172 molded at a position to snugly engage the head of the wearer when the helmet is, put on, typically at a position free of body hair, e.g. at the forehead above the eyebrow. The oximeter is of the type, e.g., as described in Figure 5b, having a source for transmitting NIR light, a detector to receive the light scattered from tissue such as brain tissue and a barrier to engage the head between the light source and the detector to prevent light traveling laterally between source and detector through subcutaneous layers.
Preferably, the oximeter in the helmet includes a control circuitry on a miniature chip and preferably circuitry and/or software are provided for determining the rate of change of oximetry readings and for comparing the rate of change to a standard.

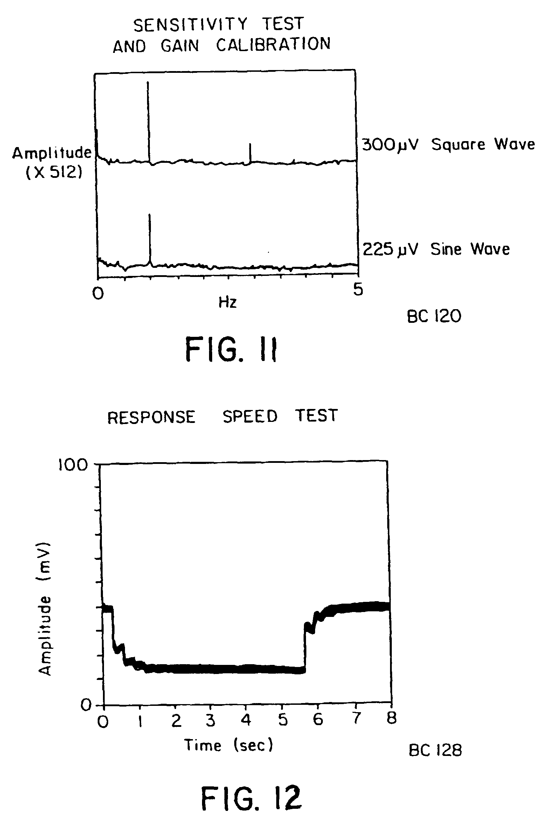

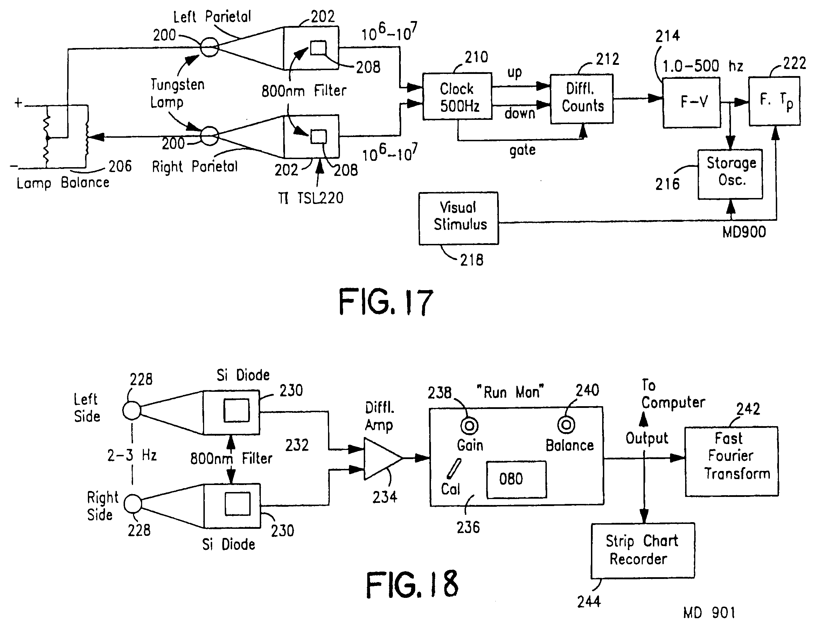

The cognition spectrophotometer system, in one embodiment, measures the low frequency and power spectra of fluctuations of absorbances attributed to the blood concentration changes in the frontal region of the brain. The low frequency recurrences of brain activity are linked to blood concentration increases and are detected in human subjects with an optical device. The spectrophotometer employs wavelengths of light sensitive to oxygenation/deoxygenation of hemoglobin in the red region of the spectrum, i.e., absorbency changes at 760 nm are balanced against those at 850 nm in equal proportions. The difference in the absorbency changes is highly sensitive to the oxygenation/deoxygenation of hemoglobin (HbO₂/Hb) and is insensitive to the changes of blood concentration. The sum of the absorbency changes of these two wavelengths is sensitive to the blood concentration changes and insensitive the hemoglobin to oxygenation/deoxygenation of hemoglobin changes (approximately one-half of the 760 nm signal is added to the 850 nm signal). The data reported in Figures 11-16 have been obtained in the sum mode and the time dependence of changes of blood concentration has been measured. An alternative method to the sum of two wavelengths is to use a single wavelength apparatus, shown in Figures 17 and 18, for example, 800 nm which is an isosbestic point in the Hb/HbO₂ spectrum. Other wavelengths can be also used, for example, 950 nm which is sensitive to water absorption, or wavelengths sensitive to endogenous or exogenous pigments.

The cognition spectrophotometer of Figure 1 was tested on several human subjects who were juniors or seniors from Central and Girls High Schools in West Philadelphia. The subjects were exposed to visual stimulation by a series of abstractions composed of analogies taken from SAT examinations. (All studies were conducted under IRB-approved protocol #M1025 for acquiring optical signals from the forehead of human subjects at rest and under test. Parental approval was obtained for those subjects under 18 years of age.) Sixty of these abstractions were displayed for an intended time of 11 minutes corresponding to 16 iterations of the Fourier transform oscilloscope 256 of the display module (Figure 1). The advance of one analogy to the next was dictated by the subject's conclusion that the analogy has been understood. The analogies presented to the subjects in this study were intended to simulate associative responses. The analogies continued within the 11-minute interval as long as the subject felt that he/she was adequately able to concentrate on them. If the subject's attention was diminished due to fatigue, etc., the test was terminated. Usually more than 11 out of the 16 iterations of the Fourier transformations were obtained and often the full 16 were obtained. At this point the subject was told to "rest".

No analogies were presented during the "rest" intervals. The Fourier transformations of the optical data for two successive 11-minute intervals were recorded and subtracted and were entitled "rest"-"rest" (Figure 15).

The sensor module used in this study consisted of two tungsten flashlight bulbs placed 4 cm each from the two silicon diodes (4 mm X 10 mm), each equipped with an interference filter transmitting a 10 nm wide band centered at 760 nm and 850 nm. Thus, this system provided two photon migration paths with input-output separation of 4 cm. A preamplifier coupled the optical signals to an amplifier unit that took the sum and difference of the signals suitably corrected as described above. The tungsten lamps were pulsed at 3 Hz so that absorbance measurements were time shared with background measurements and the one was subtracted from the other to provide background light correction via sample and hold techniques for the difference circuit.

In a response speed test, the output of the filtered sum response reached 70% in 1 sec, as shown in Figure 12. The output signal was directly connected to the Fast Fourier transformer Tupe 440 (Nicolet) which was used in the DC-coupled mode on the 200 millivolts scale. The conditions were set for 16 iterations in the 0-5 Hz scale for a total interval of data acquisition of 10 min. The recording sensitivity was 220 µv/cm at X512 gain (Figure 11) and 440 µv/cm at x256 gain. The noise level of Figures 11-15 was about 20 mV. The total signal was 115 mv and the noise level was 0.2%. The full scale sensitivity was 1.0% of absorbance change at x512 and 2.0% at x256. Figure 11 illustrates the response of the Fourier transform oscilloscope to 300 µV peak-to-peak square wave (top trace) and 225 µV sine wave (lower trace), wherein 16 iterations at x512 gain were used.

Referring to Figure 12, the response of the DC amplifier to a square wave reaches 70% in 1 sec. Thus, the system is responsive only to frequency components in the 0-3 Hz region and records the recurrence of these components. Static experiments carried out simultaneously indicate that the sum of the peaks of the test trace corresponds to an increase of blood concentration of approximately 5% of the total signal.

The ideal study control would repeat every feature of the study except the specific changes due to recognition of the analogies given to the subjects.
Thus, the study was repeated with two intervals of "rest" of duration equal to that of the "rest"-"test" study. The "test"-"test" was not considered appropriate because the double duration of the "test" might have led to accommodation in the responses, and because the response to different "tests" could be different. This study had the sensor module placed in one location during the test interval; however, mapping of the "test" response by locating the sensor module in different locations on the exterior of the head is feasible.

Each recurrent signal is indicated by a peak at a particular frequency (Hz) in the illustrations. Peaks present in the "rest" recording are usually related to arterial pulse and related frequencies. Additional frequencies are usually observed in the "test" interval, and the Fournier transforms are readily subtracted by the instrument ("test"-"rest"). Some recurrent frequencies may also contribute to the increasing intensity of the spectra in the low frequency region. Peak size, as well as the 1/f noise is increasing as the frequency decreases.

Both the "test" and "rest" signals contain a signal of the arterial pulse of the brain tissue. At "rest" and "test", signals at 1-2 Hz and related harmonics are often observed. At "rest", a low frequency component is detected at various locations on the forehead and tracks closely the frequency of arterial pulse as detected on the wrist. However, these signals nearly completely cancel in the "rest"-"rest" difference Fourier and are thus attributed to the arterial pulse in the brain tissue. Occasionally, the "test"-"rest" shows a difference signal due to altered arterial pulse rate during the "test".

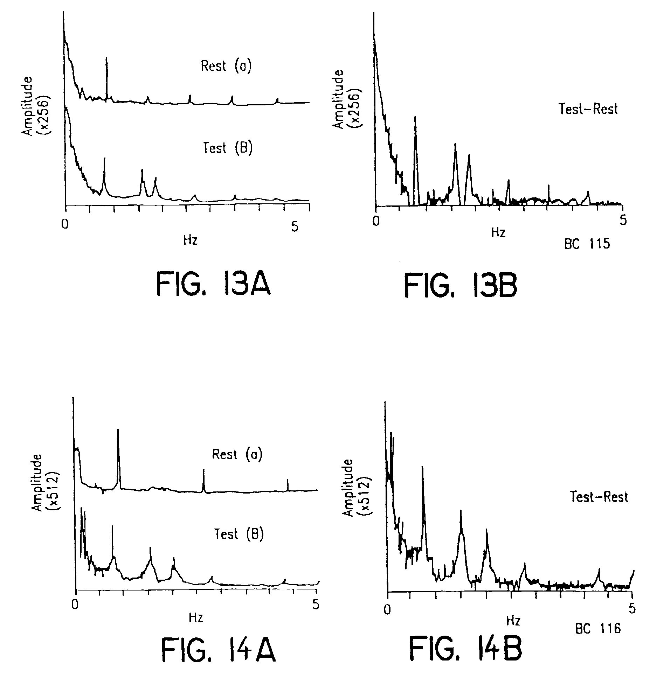

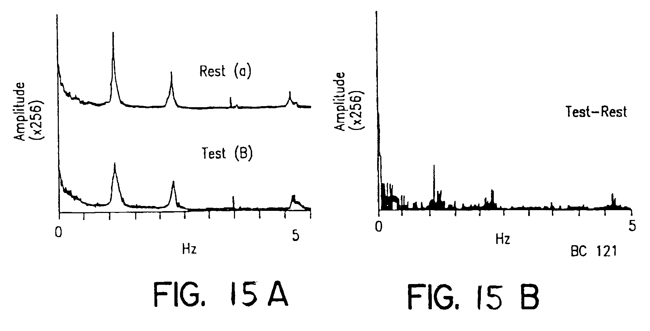

Referring to Figures 13 and 14, the Fourier transform spectrum of the "test" interval and the "rest" interval of a female subject (U) are displayed as traces b and a, respectively. 16 out of the 16 scans were obtained (study 24, Table I). The frequency scale is 0-5 Hz, the amplitude scale represents a gain of x256. The subject's heart rate was 81 BPM (1.3 Hz). However, in the "rest" spectrum, the predominant peak is at 0.8 Hz, approximately 2% of the total signal. Small, sharp peaks appear at 1.7, 2.7, 3.5 and 4.7 Hz. Similar peaks of similar amplitude appear in the "test" study and are thus not "activity related". However, new large peaks appear at 1.6 and 1.8 Hz in the test spectrum.

The difference of the two Fourier transforms designated "test"-"rest" shows a recurrence of signals at particular frequencies associated with presentation of analogies. There is a preponderance of the 0.8, 1.6 and 1.8 Hz peaks and a small contribution of a 2.8 Hz peak. The largest peak corresponds to 1% of the total signal. Of interest from the theoretical standpoint is a large component of 1/f noise in the "test"-"rest" diagram suggesting that recurrence at the very low frequencies is less probable than at the higher frequencies. In a repetition of this study (#25), a 2.3 Hz peak was observed (see Table I) and the doublet peaks at 1.7 and 1.9 Hz were again observed in "test"-"rest".

Another frequency spectrum of a female subject (B) is shown in Figure 14 where the rest spectrum shows a strong peak at 0.8 Hz, presumably due to the heart rate, and a second peak at 2.7 Hz. After 7 iterations, the subject spontaneously ceased the study. The Fourier transform of the "test"-"rest" spectrum contains broad peaks at 0.8, 1.5, 2.0, 2.7 and a peak at 4.3 Hz. The largest peaks exceed 1% of the total signal. The 1/F noise observed in the spectra appears to contain an unresolved peak at 0.2 Hz.

Referring to Figure 15, frequency analysis of two "rest" spectra of a subject U shows somewhat stronger peaks than in the "rest" study of Figure 18 at 1.2 and 2.3 Hz with a small peak at 4.6 Hz. In the "rest"-"rest" traces, only small broad peaks are observed. There are small differences that are attributed to variations in the arterial pulse and its higher frequency components of amplitude and frequency that differ in the first "test" as compared to the second. This is confirmed in Tests 27 and 28. In no case was a new distinctive peak observed as in "test"-"rest" studies. Table I summarizes 31 tests of 9 individuals (6 females, 3 males) over a six-week period. Column F lists the frequencies observed at "rest", Column G, the frequencies during the "test" interval (except Cases 8, 27, 28). Column H gives the recurrence of frequencies appearing in the substraction of "test" and "rest" in the Fourier transform. A high consistency is indicated by our observation of recurring frequencies in 24 out of 28 tests (85%) (all but four tests 2,3,9, and 24). In No. 24, increased 1/f noise was observed (Column H). Tests 8, 27, 28 were controls in which "rest"-"rest" intervals were subtracted.

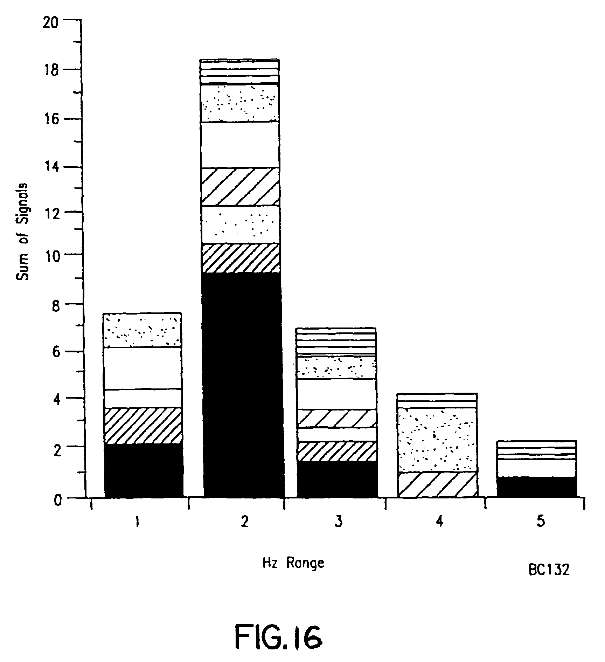

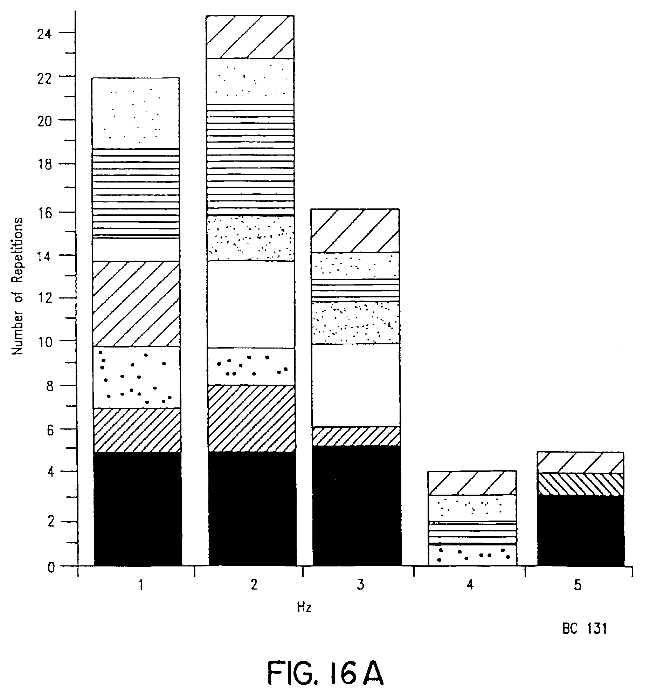

Referring to Figure 16, the histogram displays the recurrence of frequencies in the range 0-4 Hz taken from Column H of Table I. Each individual's frequencies are cross hatched. There appears to be a preponderance of 1-2 Hz signals taking into account that the frequency range is limited by commingling with 1/f noise at the low end, and the frequency response of the instrument diminishes signals above 2.5 Hz.

The intensity value at the particular frequency gives the power spectrum of Figure 16a for 1 Hz intervals. The largest peak is at 1.5-2.5 Hz. In both cases, the stippling of the chart identifies the nine subjects.

A more detailed plot of the data of Table I, including those that exhibited three or more frequencies, indicated that this ensemble exhibited equal maxima at the harmonic frequencies 0.8 and 1.6 Hz and approximately equal peaks at 1.2, 2.6 and 4.2 Hz respectively, a rough approximation to harmonics of frequencies of 0.08 and 1.2 Hz. However, there was no peak at 3.0, 2.1 Hz at the exact harmonic frequencies.

The cognition spectrophotometer system, used in this trial, utilizes only the sum of the 760 nm and 850 nm signals. The isosbestic point in the Hb/HbO₂ spectrum is approximately at 800 nm, and thus the balanced sum of Hb/HbO₂ absorbances at 760 and 850 nm measures the blood concentration. Hb/HbO₂ is the principal absorber at these wavelengths since the signal is over 20 times that of cytochrome aa₃. The fact that no significant results were obtained in the difference recording suggests that the oxygenation/deoxygenation of the frontal lobe tissue was not a predominant effect in these studies. Changes of light scattering are not separated from absorbance changes when using continuous light, and would be expected to uniformly affect both 760 and 850 nm.

Furthermore, results of the present trial are compatible with other studies using PET, SPEC, and MRI which also suggest existence of blood flow changes during brain stimulation and the fact that the arterial pulse is the strongest signal in both "rest" and "test"; this supports the interpretation attributing the measured signal to blood concentration changes.

It is expected that the metabolic activities induced by brain stimulation contain low frequency component signals. The cognition spectrophotometer system of Figure 1 records repetitive fluctuations in the optical signal in a 10 min stimulus interval. Thus, the employed method requires not only a blood concentration increase during stimulation, but also periodic repetition of the increase within the tissue volume optically sampled. Such increases need not be repeated at identical locations but may have a spatiotemporal distribution. A 4 cm separation of input/output gives a mean penetration of 2 cm and a tissue volume of roughly 5 ml within which the repetitive response is observed.

Our narrow band width and relatively short iteration time (<11 min.) in this trial was set by the characteristics of an available instrument, and the detected spectrum may be only a portion of the total frequency spectrum. As shown in Figure 17, a much faster instrument can be also used. The limits to the bandwidth of optical studies are set both by signal-to-noise and by the intrinsic relaxation time of photon migration in the brain.

In the above described trial only a crude localization of the response was employed by placement of the input and output ports. The optical system was designed to respond optimally to blood concentration changes (in a volume of about 3-5 ml) in the frontal/temporal cortex, and the analogies were designed to stimulate activity in the frontal region. When left and right sides of the forehead were compared, the success rate of new peaks in the Fourier transform was two times greater in the left side.

An important aspect of future development of the optical methods is the rate at which data may be accumulated. The travel time of photon migration over a path length of one meter is 23 ns with a 4 cm input/output separation. The signal to noise ratio will determine the number of iterations of the test, which in these preliminary studies, was approximately 50 over a period of 11 minutes.

The photon migration kinetics are responsive to both light scattering and light absorption. Whilst absorption has been stressed in these studies, light scattering is much closer to the primary events of neuronal/axonal response than to the blood flow/blood concentration change, both in anatomy and in time.

In this trial, the low frequency recurrence of changes of blood concentration was measured in portions of the brain that are approximately 2 cm deep from the surface of the skull of the subjects. Application to other stimuli (for example, visual, acoustic, sensorimotor) that affect function in other regions of the brain may be possible. (Visual stimulation registered in the visual cortex was not used in the present trial because dense hair covered the surface of the back of the head in the population studied.) It is expected that the recurrence frequencies which are observed in the cognitive tests would be significantly diminished in cases of neuronal deterioration. Thus, the cognition spectrophotometer system enables simple non-invasive and rapid testing of cognitive function of different regions of the brain.

The cognition spectrophotometer system is designed to provide objective evaluation of functional activity of the brain with a simple and inexpensive device. The system enables a wide range of studies of the brain activity in responding to appropriate tasks and presents an alternative to several expensive techniques (for example, MEG and EEG) or ones that involve radioactive techniques. Thus, screening for neuronal deterioration and/or deterioration of brain function can be done conveniently and continuously.

In another embodiment, shown in Figures 17 and 18, the cognition spectrophotometer simultaneously records lateralized potential difference of two brain hemispheres. It has been suggested that lateralized readiness potential can measure electrical brain activity that is related to preparation of movement. This measure has been used to illuminate pre-setting processes that prepare the motor system of the brain for action. The lateralized potential also demonstrates the presence of transmission of partial information in the cognitive system and identifies processes responsible for the inhibition of responses.

The cognition spectrophotometer, shown in Figures 17 and 18, measures the lateralized difference potential optically by measuring increased blood concentration in localized regions of the brain that are activated by the lateralized readiness potential. The spectrophotometer simultaneously records and compares optical signals from both hemispheres. The detected data are subtracted in real time, the difference signal is recorded as an analog signal via an A/D converter and a computer manipulates the data and takes the Fourier transform of the data. The lateralized difference potential is measured as blood concentration changes using 800 nm light; however, light of other wavelengths sensitive to other constituents of the brain tissue can be used.

For high frequency measurements, the cognition spectrophotometer can use a fast lateralization detector, shown in Figure 17. The detector has two tungsten light sources 200 placed on the left and right parietal bones and two light-to-frequency converters 202 placed also or the parietal bones at a distance of 4 centimeters from the sources. The intensity of each lamp 200 is regulated differentially by a lamp balance 206 in order to achieve the same light intensity output from the two light sources. An 800 nm interference filter 208 is placed on each silicon detector 202. The silicon light-to-frequency converters are adjusted by the frequency light intensity control to output the same frequency in the range of 10⁶ to 10⁷ Hz.

The fast lateralization detector measures a frequency difference between the two silicon detector converters 204. The signal of each detector is routed to a differential counter 212 using a 50 Hz clock 210. If the differential count is zero, both detectors are outputting the same frequency signal. If there is a difference in the signals detected at the two detectors, the differential count is not zero. The differential signal is processed by a frequency-to-voltage converter 214. Converter 214 operates in a low frequency range and detects frequency differences. The voltage output is Fourier transformed, stored and analyzed in view of a visual stimulus 218 or other brain activity stimulus.

Referring to Figure 18, in another preferred embodiment the cognition spectrophotometer utilizes a narrow band lateralization detector which is sensitive to the low frequency changes. Tungsten lamps 228 placed on the parietal bone are flashed at frequencies on the order of 2 to 3 Hz in order to provide for correction of the dark current. Each detector 230 has an 800 nm interference filter 232. A differential amplifier 234 is used to measure difference in the light intensity arriving at the two detectors. The output of the differential amplifier is coupled to the "RunMan" control unit, a modified version of the control unit shown in Figures 7 and 8. Balance 240 is set so that the two signals are balanced at zero millivolts. The output signal is connected to the fast Fourier transformer 242, recorded on the strip chart recorder 244 or sent to a computer. The lateralization detector of Figure 18 measures differential low frequency fluctuations of absorbances attributed to the brain blood concentration changes in different respective regions of the two brain hemispheres. The localization of the radiation to the appropriate region of the brain hemisphere is achieved by selecting proper location and separation of the input and output ports of the source-detector pair (Figures 1, 17, 18 and 19). For example, as described above, 4 cm separation of the source and detector on the exterior of the head gives a penetration of about 2 cm of the banana-shaped migration path of the radiation.

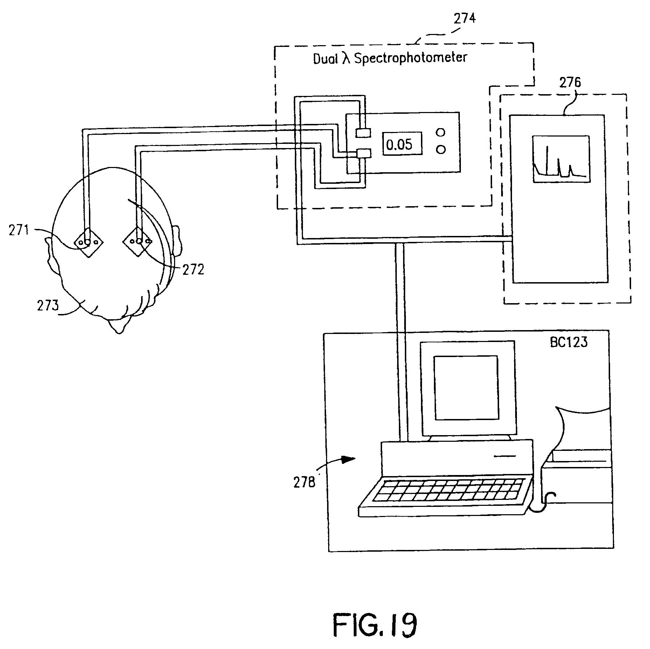

Referring to Figure 19, another preferred embodiment of the present invention is a differential spectrophotometer system for in vivo examination of a tissue of a human by measuring changes in electromagnetic radiation migrated in a path of two localized tissues of interest. The first tissue of interest, used to collect reference data, is expected to have normal physiological properties. The second tissue of interest is a tissue expected to have regions of pathological or pathophysiological changes, for example, a tumor or bleeding. An approximate localization of the tissue of interest having pathological or pathophysiological changes can be determined by diagnostic assessment of the subject, for example, by a neurologic examination, evaluation of the symptoms, etc.. In absence of any pathophysiological changes both the first and second localized tissues of interest (for example, regions of the left and right hemisphere of the brain, left and right breast, or left and right arm) are expected to produce nearly identical signals of photon migration. On the other hand, if a region of an organ, for example, the right hemisphere of the brain, has a tissue of abnormal pathophysiological state, the tissue is expected to produce a signal which differs from the signal of the normal tissue of the left hemisphere; thus, the differential signal will be non zero.

Referring to Figure 19, the differential spectrophotometer system comprises the following five modules: two sensor modules 271 and 272, a control circuit module 274, an analyzer/display module 276, and a computer/printer module 278. Each of the two sensor modules 271 and 272, placed on the subject's head 273, comprises at least one source and detector as shown in Figures 2 to 5c, 17, 18. The operation of each sensor module is governed by control circuit module 274 which controls the radiation emission and detection, collects the data and sends the data to analyzer/display module 276 and to computer/printer 278. The operation of the whole cognition system is controlled by a computer of computer/printer module 278.

The differential spectrophotometer introduces electromagnetic radiation of at least one selected wavelength (for example, 800 nm) into the brain simultaneously at a first and second input port of the 271 and 272 sensor modules. Both the first input port and the second input port are placed at a first selected location and a second selected location on the exterior of the head, respectively. Two detectors, placed at a first and second location on the exterior of the head, are simultaneously detecting radiation that migrated along the banana-shaped paths in the two brain hemispheres from the first and second input ports to the first and second output ports, respectively. Control module 274 collects the detector signals, corrects for the noise signals and sends the corrected signals to analyzer/display module 276 and to computer/printer module 278. Control module 274 can also create a differential signal, as described in Figures 22 and 23.

The system processes the signals of the two detectors and evaluates the processed data to determine whether the radiation migrated in a tissue of abnormal physiological or pathophysiological properties.

In another application, the differential spectrophotometer system can be used to determine physiological or pathophysiological properties of a breast tissue. Here, one sensor is placed on one breast and the other sensor on the other breast. Similarly as for the brain, breast tissue is examined by comparing the two signals of the radiation that migrated in the two paths in the respective breast.

The lateralized detection technique that simultaneously measures photon migration in two migration paths (Figures 17, 18, and 19) achieves better signal-to-noise ratio than the technique which detects signals from only one migration path (Figure 15). Some lateralized events involve only blood volume changes or only blood concentration changes; thus, the system can probe the tissue using radiation of only one selected wavelength. Other lateralized events may result in oxygenation/deoxygenation changes; thus, the system uses radiation of at least two wavelength (for example, 760 nm and 850 nm) coupled into an organ alternatively. In this case, each sensor comprises either one source-detector pair adapted to operate at at least two selected wavelengths, or two wavelength specific source-detector pairs. Signals of each wavelength obtained from the two sensors are again differentially manipulated to determine desired tissue properties. As described above, the localization of the radiation is controlled by placement and position of each source and detector.

One of ordinary skill in the art will appreciate that the present invention is not limited to the particular embodiments described in detail. Modifications to the circuitry disclosed, and other aspects of the spectrophotometer configurations disclosed, as well as other modifications to the physical arrangement of the present apparatus will be obvious to those of ordinary skill. Further, the present invention is not limited to any of the uses described herein.

## Claims

1. A method of deriving data signals useful for determining a physiological or pathophysiological property of a localized region of human tissue of a subject, the method comprising the steps of: providing at least one light source, a first input port and a second input port for introducing electromagnetic radiation from said at least one light source to human tissue, and at least one light detector adapted operatively to detect, at a first detection port and a second detection port, radiation that has migrated in the tissue; the method being characterized in that said first input port and said second input port are arranged to introduce electromagnetic radiation of a selected wavelength from said at least one light source to said human tissue; in that said first input port and said first detection port are placed on the subject separated by a selected distance, at selected locations relative to the tissue to be examined, said distance and locations defining a first tissue region of interest; in that said second input port and said second detection port are placed on the subject separated by said selected distance, at selected locations relative to the tissue to be examined, said distance and locations defining a second tissue region of interest of similar volume to said first tissue region; in that said at least one light source and said at least one detector are operated, whereby said detectors detect at the respective detection ports radiation from said light source of said selected wavelength that has migrated through the skin and tissue in the first tissue region and the second tissue region; in that signals derived by said at least one detector in response to radiation detected at said first detection port and said second detection port are processed to create first and second processed data influenced by absorption or scattering by the skin and tissue through which the radiation has migrated; and in that the first processed data is correlated with the second processed data.

2. A method according to Claim 1, wherein said step of correlating the first and the second processed data includes generating a differential signal indicative of differences in optical properties between said first tissue region and said second tissue region.

3. A method according to Claim 1 or 2, wherein said processing and correlating steps are adapted to derive data indicative of blood concentration, blood oxygenation or blood volume of the examined tissue.

4. A method according to any preceding Claim, wherein said input and detection ports are arranged for mounting on symmetrical regions of the right breast and the left breast, the right arm and the left arm, or the right leg and the left leg, respectively.

5. A method according to any of Claims 1 to 3, wherein said input and detection ports are arranged for mounting on the head, whereby to allow transcranial examination of brain tissue.

6. A method according to Claim 5, wherein said input ports and said corresponding detection ports are arranged to be located relative to one of the following: the frontal bone, parietal bone, temporal bone or occipital bone of the cranium, whereby to enable symmetrical regions of the brain to be examined.

7. A method according to Claims 5 or 6, wherein said processing and correlating steps are adapted to derive data indicating neurological conditions.

8. A method according to any preceding Claim, wherein said steps are repeated over time with said input and detection ports located in the same position, whereby to detect changes of said property.

9. A method of deriving data signals useful in transcranial examination of brain activity of a subject, the method comprising the steps of: providing a light source and an input port adapted for introducing electromagnetic radiation of a selected wavelength from the light source to brain tissue, and a light detector adapted operatively to detect, at a detection port, the introduced radiation that has migrated in the tissue; placing said input and detector ports on the head of the subject; and operating said light source and said detector to detect radiation from said light source of said selected wavelength that has migrated in a tissue region of the brain from said input port to said detection port; processing signals, derived by said detector in response to detected radiation that has migrated in the brain, to create processed data; characterized in that said operation step is performed at the same time as a brain activity is being stimulated; and in that said processed data is correlated with said stimulated brain activity.

10. A method according to Claim 9, wherein said processing and correlating steps are adapted to provide data signals indicative of cognitive function of a selected region of the brain.

11. A method according to Claims 9 or 10, further comprising repeating the said steps in the absence of stimulation of brain activity, whereby to derive processed data indicative of "no stimulation"; and correlating the data derived under stimulation and the "no stimulation" data.

12. A cognition spectrophotometer apparatus for transcranial examination of brain activity by measuring changes in electromagnetic radiation scattered and absorbed in a migration path in the brain of a subject, the apparatus comprising: a light source constructed to produce electromagnetic radiation of a selected wavelength adapted to be introduced into the brain at an input port associated with said light source and adapted to be placed at a selected location upon the exterior of the head; a light detector adapted operatively to detect, at a detection port placed at a selected location upon the exterior of the head, radiation of said selected wavelength that has migrated in the brain; and processing means, connected to said light detector and being adapted to process signals, derived by said detector in response to detected radiation from said light source that has migrated in the brain, to create processed data; the apparatus being characterised in further comprising stimulation means adapted for causing stimulation of a brain activity simultaneously with operating said light source and detector; and evaluation means adapted to derive an evaluated characteristic of brain activity by correlating said processed data with said stimulation.

13. Apparatus according to Claim 12, further characterized in that said stimulation means is adapted to cause visual stimulation, acoustic stimulation or sensorimotor stimulation.

14. Apparatus according to Claims 12 or 13, wherein said evaluation means is adapted to derive data indicative of a cognitive function of a selected region of the brain.

15. A spectrophotometer apparatus for examination of human tissue of a subject by measuring changes in electromagnetic radiation scattered and absorbed in a migration path in tissue, the apparatus comprising: a first input port and a second input port for introducing electromagnetic radiation to human tissue, and at least one light source coupled optically with the input ports for providing such electromagnetic radiation; a first detection port and a second detection port, and at least one detector coupled with said detection ports for detecting electromagnetic radiation received thereat; and a processor, including electronic circuitry, coupled to said detector to receive signals therefrom; the apparatus being characterised in that: said first input port and said second input port are each adapted for introducing electromagnetic radiation of a selected wavelength to said human tissue from said at least one light source; in that said first input port and said first detection port are constructed to maintain a first relative distance that serves to define an examination volume of a first tissue region when said ports are mounted on human tissue; in that said second input port and said second detection port are constructed to maintain a second relative distance that serves to define an examination volume of a second tissue region when said ports are mounted on human tissue; said first and second relative distances being substantially the same and said first and second tissue regions having similar volumes; said at least one detector being adapted operatively to detect at said first detection port radiation from said at least one light source that has migrated through skin and tissue in the first tissue region from said first input port to said first detection port and, at said second detection port, radiation from said at least one light source that has migrated through skin and tissue in the second tissue region from said second input port to said second detection port; and in that the processor is adapted to process said received signals to derive first and second processed data, respectively corresponding to radiation detected at said first and said second detection ports that has been influenced by absorption or scattering by the skin and tissue through which the radiation has migrated, and also to correlate said first data and said second data to provide an indication of a physiological or pathophysiological property of said tissue examined.

16. Apparatus according to Claim 15, further characterized in that there is only a single detector connected to both said detection ports.

17. Apparatus according to Claim 16, further characterized in that said detector is a differential detector adapted to compare electromagnetic radiation detected at said first and second detection ports to create processed data representing a differential signal, said differential signal providing an indication of said property.

18. Apparatus according to Claim 15, further characterized in that said processor is further adapted to compare signals of said radiation detected at said first detection port and said second detection port and to derive a differential data providing an indication of said property.

19. Apparatus according to Claim 15, further characterized in that there are two said detectors, each said detector being optically connected to one detection port.

20. Apparatus according to Claim 15, further characterized in that there are two said light sources, each said light source being connected to a respective input port.

21. Apparatus according to Claim 15, further characterized in that there is only a single light source connected to both said input ports.

22. Apparatus according to any of claims 15 to 21, further characterized in that said input and detection ports are adapted for mounting to symmetrical regions of the right breast and the left breast, the right arm and the left arm, or the right leg and the left leg, respectively.

23. Apparatus according to any of Claims 15 to 21, further characterized in that said input and detection ports are adapted to be located on one of the following: the frontal bone, parietal bone, temporal bone or occipital bone of the cranium; and said input and detection ports being maintained at a predetermined separation in order operatively to localize said migration of said radiation to a selected region of the brain.

24. Apparatus according to any of Claims 15 to 23, further characterized in that said processor is adapted to process said received signals by Fourier transformation.

25. Apparatus according to any of Claims 12 to 24, further characterized in that the (each) said light source includes a tungsten lamp or a light emitting diode.

26. Apparatus according to any of Claims 12 to 25, further characterized in that the (each) said detector includes a silicon diode with an interference filter adapted to detect radiation of said selected wavelength.

27. Apparatus according to any of Claims 15 to 25, further characterized in that the (each) said detector is a light-to-frequency convertor having an interference filter adapted to detect radiation of said selected wavelength.

28. Apparatus according to Claim 19 and Claim 27, further characterized in that said processor comprises (a) a differential counter adapted to register differential signals received from said light-to-frequency convertors; (b) clocking means adapted to route signals of said detected radiation from said light-to-frequency convertors to said differentials counter; and (c) a frequency-to-voltage converter adapted to convert signals from said differential counter.

29. Apparatus according to both Claims 20 and 24, further characterized in that said processor further comprises a Fourier transformer adapted to process the differential signals from said frequency-to-voltage converter.

30. Apparatus according to any of Claims 12 to 29, characterised in further including first and second barriers constructed and arranged to intercept superficial light migrating laterally in the skin and flesh from said input ports to the respective detection ports.

31. Apparatus according to any of Claims 15 to 21, further characterized in that said input and detection ports are adapted for mounting to symmetrical regions of the left breast and the right breast to localize said photon migration in two corresponding breast tissue regions; and in that said processor is adapted to correlate said first and said second data to provide an indication indicative of a breast tumour being present in one said region.

32. Apparatus according to any of Claims 15 to 21, further characterized in that said input and detection ports are adapted for mounting to symmetrical regions on the left and right sides of the head to localize said photon migration in two corresponding regions of the brain; and in that said processor is adapted to correlate said first data and said second data to provide an indication indicative of a brain tumour being present in one said region.

33. Apparatus according to any of Claims 15 to 21, wherein said input and detection ports are adapted for mounting to symmetrical regions on the left and right sides of the body to localize said photon migration in two corresponding regions on the left and right sides of the body; and in that said processor is adapted to correlate said first data and said second data to provide an indication indicative of bleeding in one said region.

## Patentansprüche

1. Verfahren zum Ableiten von Datensignalen, die zur Bestimmung einer physiologischen oder pathophysiologischen Eigenschaft einer lokalisierten Region oder Zone menschlichen Gewebes einer Person geeignet sind, wobei das Verfahren die folgenden Schritte aufweist:
Vorsehen von mindestens einer Lichtquelle, einer ersten Eingangsanschlusses und eines zweiten Eingangsanschlusses zum Einführen elektromagnetischer Strahlung von der mindestens einen Lichtquelle in das menschliche Gewebe und mindestens einen Lichtdetektor geeignet zum betriebsmäßigen Detektieren an einem ersten Detektionsanschluß und einem zweiten Detektionsanschluß von Strahlung, die in das Gewebe gewandert ist, wobei das Verfahren gekennzeichnet ist dadurch, daß der erste Eingangsanschluß und der zweite Eingangsanschluß derart angeordnet sind, daß sie elektromagnetische Strahlung einer ausgewählten Wellenlänge von der mindestens einen Lichtquelle zu dem menschlichen Gewebe einführen;
daß der erste Eingangsanschluß und der erste Detektionsanschluß auf der Person um einen ausgewählten Abstand voneinander angeordnet sind an ausgewählten Stellen relativ zu dem zu untersuchenden Gewebe, wobei der Abstand und die Stellen eine erste interessierende Gewebezone definieren; daß der zweite Eingangsanschluß und der zweite Detektionsanschluß auf der Person getrennt oder separiert durch den ausgewählten Abstand angeordnet sind an ausgewählten Stellen relativ zum Gewebe, welches untersucht werden soll, wobei der Abstand und die Stellen eine zweite interessierende Gewebezone mit ähnlichem Volumen wie die erste Gewebezone definieren; daß die mindestens eine Lichtquelle und der mindestens eine Detektor betrieben werden, wodurch die Detektoren an den entsprechenden Detektionsanschlüssen Strahlung von der Lichtquelle der ausgewählten Wellenlänge, die durch die Haut und das Gewebe der ersten Gewebezone und der zweiten Gewebezone gewandert sind, detektieren;
daß Signale abgeleitet durch den erwähnten mindestens einen Detektor ansprechend auf die Strahlung detektiert an dem ersten Detektionsanschluß und dem zweiten Detektionsanschluß verarbeitet werden, um erste und zweite verarbeitete Daten zu erzeugen und zwar beeinflußt durch die Absorption oder Streuung durch die Haut und das Gewebe, durch die die Strahlung gewandert ist;
und daß die ersten verarbeiteten Daten in Korrelation mit den zweiten verarbeiteten Daten gebracht werden.

2. Verfahren nach Anspruch 1, wobei der Schritt des Korrelierens der ersten und zweiten verarbeiteten Daten das Erzeugen eines Differentialsignals umfassen, welches eine Anzeige bildet für die Unterschiede der optischen Eigenschaften zwischen der ersten Gewebezone und der zweiten Gewebezone.

3. Verfahren nach Anspruch 1 oder 2, wobei die Verarbeitungs- und Korrelierungsschritte geeignet sind, um Daten abzuleiten, die eine Anzeige bilden für die Blutkonzentration, die Blutoxygenierung oder das Blutvolumen des untersuchten Gewebes.

4. Verfahren nach einem der vorherigen Ansprüche, wobei die Eingangs- und Detektieranschlüsse zur Anbringung auf symmetrischen Zonen der rechten und linken Brust bzw. des rechten und linken Arms bzw. des rechten Beines und des linken Beines angeordnet sind.

5. Verfahren nach einem der Ansprüche 1 bis 3, wobei die Eingangs- und Detektieranschlüsse zur Anbringung an dem Kopf geeignet sind, durch die transcraniale Untersuchung des Gehirngewebes gestattet ist.

6. Verfahren nach Anspruch 5, wobei die Eingangsanschlüsse und die entsprechenden Detektieranschlüsse relativ zu einem der folgenden Teile angeordnet sind: dem Frontalknochen, dem Parietalknochen, dem Temporalknochen oder Okzipitalknochen des Craniums, wodurch symmetrische Zonen des Gehirns untersucht werden können.

7. Verfahren nach Anspruch 5 oder 6, wobei die Verarbeitungs- und Korrelierschritte ausgeführt werden, um Daten abzuleiten, welche neurologische Zustände anzeigen.

8. Verfahren nach einem der vorherigen Ansprüche, wobei die Schritte über die Zeit hinweg wiederholt werden und zwar mit den Eingangs- und Detektieranschlüssen in der gleichen Position angeordnet, wodurch Änderungen in den Eigenschaft detektiert werden.

9. Verfahren zum Ableiten von Datensignalen brauchbar bei der transcranialen Untersuchung der Gehirnaktivität einer Person wobei das Verfahren die folgenden Schritte vorsieht:
Vorsehen einer Lichtquelle und eines Eingangsanschlusses geeignet zum Einführen elektromagnetischer Strahlung mit einer ausgewählten Wellenlänge von einer Lichtquelle zu dem Gehirngewebe, und eines Lichtdetektors geeignet zum betriebsmäßigen Detektieren an einem Detektionsanschluß der eingeführten Strahlung, die in das Gewebe gewandert oder gelaufen ist;
Anordnen der Eingangs- und Detektoranschlüsse am Kopf des Objektes;
Betrieb der Lichtquelle und des Detektors zum Detektieren von Strahlung von der Lichtquelle der ausgewählten Wellenlänge, die in eine Gewebezone des Gehirns gewandert ist, und zwar von dem Eingangsanschluß zu dem Detektieranschluß;
Verarbeiten der durch den Detektor abgeleiteten Signale ansprechend auf detektierte Strahlung, die in das Gehirn gewandert ist, um verarbeitete Daten zu erzeugen
dadurch gekennzeichnet, daß der Betriebsschritt zur gleichen Zeit ausgeführt wird wie die Gehirnaktivität stimuliert wird;
daß die verarbeiteten Daten mit der erwähnten stimulierten Gehirnaktivität korreliert werden.

10. Verfahren nach Anspruch 9, wobei die Verarbeitungs- und Korrelierschritte geeignet sind, um Datensignale vorzusehen, welche für eine kognitive Funktion der ausgewählten Zone des Hirns eine Anzeige bilden.

11. Verfahren nach Anspruch 9 oder 10, wobei ferner folgendes vorgesehen ist: Wiederholung der Schritte bei Abwesenheit von Stimulation der Gehirnaktivität, wodurch verarbeitete Daten abgeleitet werden, die für "keine Stimulation" eine Anzeige bilden; und Korrelieren der bei Stimulation abgeleiteten Daten mit den "keine Stimulationsdaten".

12. Kognitive Spektrophotometervorrichtung zum transcranialen Untersuchen der Gehirnaktivität durch Messen von Änderungen der elektromagnetischen Strahlung gestreut und absorbiert in einem Wanderungs- oder Fortpflanzungspfad im Gehirn einer Person, wobei die Vorrichtung folgendes aufweist: eine Lichtquelle derart konstruiert, um elektromagnetische Strahlung einer ausgewählten Wellenlänge zu erzeugen, geeignet zur Einführung in das Gehirn an einem Eingangsanschluß assoziiert mit der Lichtquelle und geeignet zur Anordnung an einer ausgewählten Stelle auf dem Äußeren des Kopfes;
ein Lichtdetektor geeignet zur betriebsmäßigen Detektion an einem Detektionsanschluß angeordnet an einer ausgewählten Stelle am Äußeren des Kopfes von Strahlung der erwähnten ausgewählten Wellenlänge, die in das Gehirn gelaufen oder gewandert ist; und
Verarbeitungsmittel verbunden mit dem Lichtdetektor und geeignet zur Verarbeitung von Signalen abgeleitet durch den Detektor ansprechend auf die detektierte Strahlung von der Lichtquelle, die in das Gehirn gewandert oder gelaufen ist, um verarbeitete Daten zu erzeugen;
wobei die Vorrichtung gekennzeichnet ist, daß ferner Stimulationsmittel vorgesehen sind, geeignet zur Bewirkung einer Gehirnaktivität gleichzeitig mit dem Betrieb der Lichtquelle und des Detektors; und
Auswertmittel geeignet zum Ableiten einer ausgewerteten Charakteristik der Gehirnaktivität durch Korrelation der verarbeiteten Daten mit der Stimulation.

13. Vorrichtung nach Anspruch 12 ferner dadurch gekennzeichnet, daß die Stimulationsmittel geeignet sind, um eine visuelle Stimulation, eine akustische Stimulation oder eine sensorimotorische Stimulation zu bewirken.

14. Vorrichtung nach Anspruch 12 oder 13, wobei die Auswertmittel geeignet sind, um Daten abzuleiten, die eine Anzeige bilden für eine kognitive Funktion einer ausgewählten Zone des Gehirns.

15. Spektrophotometervorrichtung zur Untersuchung des menschlichen Gewebes einer Person durch Messung von Änderungen der elektromagnetischen Strahlung gestreut und absorbiert in einem Fortpflanzungspfad im Gewebe, wobei die Vorrichtung folgendes aufweist:
einen ersten Eingangsanschluß und einen zweiten Eingangsanschluß zum Einführen elektromagnetischer Strahlung in das menschliche Gewebe und mindestens eine Lichtquelle optisch gekoppelt mit den Eingangsanschlüssen zum Vorsehen dieser elektromagnetischen Strahlung;
ein erster Detektoranschluß und ein zweiter Detektoranschluß und mindestens ein Detektor gekoppelt mit den Detektoranschlüssen, um elektromagnetische Strahlung, die dort empfangen wird, zu detektieren; und
ein Prozessor mit einer elektronischen Schaltung gekoppelt mit dem Detektor, um Signale davon zu empfangen;
wobei die Vorrichtung dadurch gekennzeichnet ist, daß
der erste Eingangsanschluß und der zweite Eingangsanschluß jeweils geeignet sind, zum Einführen elektromagnetischer Strahlung einer ausgewählten Wellenlänge in das menschliche Gewebe von der mindestens einen Lichtquelle;
der erste Anschluß und der erste Detektionsanschluß derart konstruiert sind, um eine erste relative Distanz aufrecht zu erhalten, die dazu dient, ein Examinations-oder Überprüfungsvolumen einer ersten Gewebezone vorzusehen, wenn die Anschlüsse an dem menschlichen Gewebe angebracht sind;
der zweite Eingangsanschluß und der zweite Detektionsanschluß derart konstruiert sind, daß eine zweite relative Distanz aufrecht erhalten wird, die dazu dient, ein Examinations- oder Überprüfungsvolumen einer zweiten Gewebezone zu definieren, wenn die Anschlüsse an menschlichem Gewebe angebracht sind;
wobei die ersten und zweiten relativen Distanzen oder Abstände im wesentlichen die gleichen sind und die ersten und zweiten Gewebezonen ähnliche bzw. gleiche Volumina besitzen;
wobei mindestens ein Detektor geeignet ist zum betriebsmäßigen Detektieren an dem ersten Detektionsanschluß von Strahlung, von der erwähnten mindestens einen Lichtquellen, d.h. von Strahlung die durch die Haut und das Gewebe in der ersten Gewebezone gelaufen ist von dem ersten Eingangsanschluß zu dem ersten Detektionsanschluß, und wobei der zweite Detektionsanschluß Strahlung detektiert von der mindestens einen Lichtquelle, die durch die Haut und das Gewebe in der zweiten Gewebezone gewandert ist von dem zweiten Eingangsanschluß zu dem zweiten Detektionsanschluß; und
der Prozessor geeignet ist, die empfangenen Signale zu verarbeiten, erste und zweite verarbeitete Daten zu erhalten, die jeweils der Strahlung entsprechen, detektiert an den ersten und zweiten Detektionsanschlüssen, die durch Absorption oder das Streuen durch die Haut und das Gewebe beeinflußt ist, durch welche die Strahlung gelaufen ist, und ebenfalls zur Korrelation der ersten Daten und der zweiten Daten, um eine Anzeige vorzusehen für eine physiologische oder pathophysiologische Eigenschaft oder Charakteristik des untersuchten Gewebes.

16. Vorrichtung nach Anspruch 15 ferner dadurch gekennzeichnet, daß nur ein einziger Detektor mit beiden Detektoranschlüssen verbunden ist.

17. Vorrichtung nach Anspruch 16 ferner dadurch gekennzeichnet, daß der Detektor ein Differentialdetektor ist, der geeignet ist, um die elektromagnetische Strahlung detektiert an den ersten und zweiten Detektionsanschlüssen zu vergleichen, um verarbeitete Daten zu erzeugen, welche ein Differential-oder Differenzsignal repräsentieren, welches eine Anzeige der erwähnten Eigenschaft oder Charakteristik vorsieht.

18. Vorrichtung nach Anspruch 15 ferner dadurch gekennzeichnet, daß der Prozessor ferner geeignet ist, Signale der Strahlung zu vergleichen detektiert an dem ersten Detektionsanschluß und dem zweiten Detektionsanschluß, um Differential- oder Differenzdaten zu erhalten, welche eine Anzeige der Eigenschaft oder Charakteristik vorsehen.

19. Vorrichtung nach Anspruch 15 ferner dadurch gekennzeichnet, daß zwei Detektoren vorgesehen sind, wobei jeder Detektor optisch mit dem einen Detektionsanschluß verbunden ist.

20. Vorrichtung nach Anspruch 15 ferner dadurch gekennzeichnet, daß zwei Lichtquellen vorhanden sind, wobei jede Lichtquelle mit einem entsprechenden Eingangsanschluß verbunden ist.

21. Vorrichtung nach Anspruch 15 ferner dadurch gekennzeichnet, daß nur eine einzige Lichtquelle mit beiden Eingangsanschlüssen verbunden ist.

22. Vorrichtung nach einem der Ansprüche 15 bis 21 ferner dadurch gekennzeichnet, daß die Eingangs- und Detektionsanschlüsse geeignet sind zur Anbringung an symmetrischen Zonen der rechten und linken Brust, des rechten und linken Arms bzw. des rechten Beins und linken Beins.

23. Vorrichtung nach einem der Ansprüche 15 bis 21 ferner dadurch gekennzeichnet, daß die Eingangs- und Detektionsanschlüsse geeignet sind, um an einem der folgenden Teile angeordnet zu sein;
dem Frontalknochen, dem Parietalknochen, dem Temporalknochen oder Okzipitalknochen des Craniums; und
wobei die Eingangs- und Detektionsanschlüsse auf einem vorgeschriebenen Abstand oder Trennung gehalten werden, um die Wanderung oder den Lauf der Strahlung zu einer ausgewählten Zone des Gehirns betriebsmäßig zu lokalisieren.

24. Vorrichtung nach einem der Ansprüche 15 bis 23 ferner gekennzeichnet dadurch, daß der Prozessor geeignet ist, die empfangenen Signale durch Fouriertransformation zu verarbeiten.

25. Vorrichtung nach einem der Ansprüche 12 bis 24 ferner dadurch gekennzeichnet, daß (jede) Lichtquelle eine Wolframlampe oder eine Licht emitierende Diode aufweist.

26. Vorrichtung nach einem der Ansprüche 12 bis 25 ferner dadurch gekennzeichnet, daß (jeder) Detektor eine Siliziumdiode aufweist mit einem Interferenzfilter, geeignet zur Detektion von Strahlung der ausgewählten Wellenlänge.

27. Vorrichtung nach einem der Ansprüche 15 bis 25 ferner dadurch gekennzeichnet, daß (jeder) Detektor ein Licht-zu-Frequenzumwandler ist mit einem Interferenzfilter, geeignet zum Detektieren von Strahlung der ausgewählten Wellenlänge.

28. Vorrichtung nach Anspruch 19 und 27 ferner dadurch gekennzeichnet, daß der Prozessor folgendes aufweist:
(a) ein Differentialzähler geeignet zur Aufzeichnung von Differentialsignalen empfangen von den Licht-zu-Frequenzumwandlern;
(b) Taktmittel geeignet, um Signale der detektierten Strahlung von den Licht-zu-Frequenzumwandlern zu dem Differentialzähler zu leiten;
(c) ein Frequenz-zu-Spannungsumwandler, geeignet zum Umwandeln der Signale von dem Differentialzähler.

29. Vorrichtung nach Anspruch 20 und 24 ferner dadurch gekennzeichnet, daß der Prozessor ferner einen Fouriertransformator aufweist, geeignet zur Verarbeitung der Differentialsignale von dem Frequenz-zu-Spannungsumwandler.

30. Vorrichtung nach einem der Ansprüche 12 bis 29 dadurch gekennzeichnet, daß ferner erste und zweite Barrieren konstruiert und angeordnet sind, um die oberflächliche Lichtwanderung seitlich oder lateral in der Haut und im Fleisch von den Eingangsanschlüssen zu den entsprechenden Detektionsanschlüssen zu unterbinden.

31. Vorrichtung nach einem der Ansprüche 15 bis 21 ferner dadurch gekennzeichnet, daß die Eingangs- und Detektieranschlüsse geeignet sind zur Anbringung an symmetrischen Zonen der linken und rechten Brüste, um die Photonenwanderung in zwei entsprechende Brustgewebezonen zu lokalisieren; und daß der Prozessor geeignet ist, die ersten und zweiten Daten zu korrelieren, um eine Anzeige vorzusehen, welche anzeigt, ob ein Brusttumor in einer der Zonen vorhanden ist.

32. Vorrichtung nach einem der Ansprüche 15 bis 21 ferner dadurch gekennzeichnet, daß die Eingangs- und Detektionsanschlüsse geeignet sind zur Anbringung an symmetrischen Zonen an den linken und rechten Seiten des Kopfes, um die erwähnte Photonenwanderung in zwei entsprechenden Zonen des Gehirns zu lokalisieren; und daß der Prozessor geeignet ist, die ersten und zweiten Daten zu korrelieren, um eine Anzeige vorzuziehen, welche angibt, ob in einer Zone ein Gehirntumor vorhanden ist.

33. Vorrichtung nach einem der Ansprüche 15 bis 21 wobei die Eingangs- und Detektionsanschlüsse geeignet sind zur Anbringung an symmetrischen Zonen an den linken und rechten Seiten des Körpers, um die Photonenwanderung in zwei entsprechenden Zonen der linken und rechten Seiten des Körpers zu lokalisieren; und daß der Prozessor geeignet ist, die ersten und zweiten Daten zu korrelieren, um eine Anzeige vorzusehen, welche ein Bluten in einer der Zonen anzeigt.

## Revendications

1. Procéde pour générer des signes de données utiles pour déterminer une propriété physiologique ou physiopathologique d'une zone localisée du tissu humain d'un patient, ce procédé consistant à utiliser au moins une source lumineuse, une première et une seconde borne d'entrée pour introduire un rayonnement électromagnétique provenant de cette (ces) source(s) de lumière dans le tissu humain et au moins un détecteur fonctionne de lumière pour détecter, au niveau d'une première et d'une seconde borne de détection, le rayonnement ayant migré dans le tissu, ce procédé étant caractérisé en ce que ces première et seconde bornes d'entrée sont conçues pour introduire dans le tissu humain un rayonnement électromagnétique d'une longueur d'onde choisie provenant de cette (ces) source(s) de lumière ; en ce que les première et seconde bornes de détection disposées sur le patient sont séparées par une distance choisie, à des emplacements définis par rapport au tissu humain à examiner, cette distance et ces emplacements délimitant une première zone de tissu à examiner ; en ce que la seconde borne d'entrée et la seconde borne de détection placées sur le patient sont séparées par cette distance choisie, à des emplacements définis par rapport au tissu à examiner, cette distance et ces emplacements délimitant une seconde zone de tissu intéressante dont le volume est similaire à celui de la première zone ; en ce que cette (ces) source(s) de lumière et au moins un (ou un de ces) détecteur(s) sont actionnés, ce ou ces détecteurs décelant ainsi au niveau des bornes respectives le rayonnement de longueur d'onde donnée provenant de la source de lumière et ayant migré au travers de la peau et du tissu dans la première et la seconde zone de tissu ; en ce que les signaux extraits de ce(s) détecteur(s) à la suite de la détection du rayonnement au niveau de la première et de la seconde borne de détection sont traités pour engendrer des premières et secondes données traitées fonction de l'absorption ou de la diffusion par la peau et le tissu au travers desquels le rayonnement a migré ; et en ce que les premières données traitées sont corrélées avec les secondes.

2. Procédé selon la revendication 1, dans lequel l'étape de corrélation des premières et secondes données traitées comprend la production d'un signal différentiel indicatif des différences de propriétés optiques entre la première zone de tissu et la seconde.

3. Procédé selon la revendication 1 ou la revendication 2, dans lequel les étapes de traitement et de corrélation sont adaptées pour générer des données indiquant la concentration sanguine, l'oxygénation sanguine ou le volume sanguin du tissu examiné.

4. Procédé selon l'une quelconque des revendications précédentes, dans lequel les bornes d'entrée et de détection sont conçues pour être placées sur des zones symétriques du sein droit et du sein gauche, du bras droit et du bras gauche ou de la jambe droite et de la jambe gauche.

5. Procédé selon l'une quelconque des revendications 1 à 3, dans lequel les bornes d'entrée et de détection sont conçues pour être placées sur la tête et permettre un examen du tissu cérébral à travers le crâne.

6. Procédé selon la revendication 5, dans lequel les bornes d'entrée et les bornes de détection correspondantes sont conçues pour être placées sur l'une des parties du corps suivants : l'os frontal, l'os temporal, l'os occipital et le crâne, de manière à permettre à des zones symétriques du cerveau d'être examinées.

7. Procédé selon la revendication 5 ou la revendication 6, dans lequel les étapes de traitement et de corrélation sont adaptées pour générer des données indiquant les conditions neurologiques.

8. Procédé selon l'une quelconque des revendications précédentes, dans lequel les étapes sont répétées dans le temps, les bornes d'entrée et de détection restant situées en même position de manière à permettre de détecter des modifications de ladite propriété.

9. Procédé pour générer des signaux de données utiles aux examens de l'activité cérébrale d'un patient à travers son crâne, ce procédé consistant à utiliser une source lumineuse et une borne d'entrée adaptée pour introduire dans le tissu humain un rayonnement électromagnétique de longueur d'onde choisie provenant de cette source lumineuse et un détecteur fonctionne de lumière pour détecter, au niveau d'une borne de détection, le rayonnement ayant migré dans le tissu ; placer ces bornes d'entrée et de détection sur la tête du patient ; et actionner la source lumineuse et le détecteur pour détecter le rayonnement de longueur d'onde choisie provenant de cette source et ayant migré dans la zone de tissu du cerveau de la borne d'entrée vers la borne de sortie ; traiter les signaux extraits par ce détecteur pour générer des données traitées ; caractérisé en ce que l'étape consistant à actionner la source et le détecteur est simultanée avec la stimulation de l'activité du cerveau ; et en ce que les données traitées sont corrélées avec l'activité stimulée du cerveau.

10. Procédé selon la revendication 9, dans lequel les étapes de traitement et de corrélation sont adaptées pour fournir des signaux de données indiquant la fonction cognitive d'une zone choisie du cerveau.

11. Procédé selon la revendication 9 ou la revendication 10, comprenant en outre la répétition de ces étapes sans stimulation de l'activité du cerveau de manière à extraire des données traitées indiquant la 〈〈 non stimulation 〉〉 ; et la corrélation des données extraites sous stimulation et 〈〈 sans stimulation 〉〉.

12. Dispositif de spectrophotométrie cognitive pour l'examen à travers du crâne de l'activité cérébrale par mesure des modifications d'un rayonnement électromagnétique diffusé et absorbé sur un trajet de migration dans le cerveau d'un patient, ce dispositif comprenant : une source lumineuse conçue pour produire un rayonnement électromagnétique d'une longueur d'onde choisie adapté pour être introduit dans le cerveau au niveau d'une borne d'entrée associée à cette source lumineuse cette borne étant adaptée pour être disposée à un emplacement choisi à l'extérieur de la tête ; un détecteur fonctionnel de lumière pour détecter, au niveau d'une borne de détection disposée à un emplacement choisi à l'extérieur de la tête, le rayonnement de longueur d'onde choisie et ayant migré dans le cerveau ; et des moyens de traitement, reliés à ce détecteur de lumière et adaptés pour traiter des signaux, extraits par ce détecteur dans le cerveau, pour générer des données traitées ; ce dispositif étant caractérisé en ce qu'il comprend en outre un moyen adapté pour stimuler l'activité d'un cerveau simultanément avec l'étape consistant à actionner la source lumineuse et le détecteur ; et un moyen adapté pour fournir l'évaluation d'une caractéristique de l'activité cérébrale en corrélant ces données traitées avec ladite stimulation.

13. Dispositif selon la revendication 12; caractérisé en outre en ce que le moyen de stimulation est adapté pour générer une stimulation visuelle, une stimulation acoustique ou une stimulation sensori-motrice.

14. Dispositif selon les revendications 12 ou 13, dans lequel le moyen d'évaluation est adapté pour fournir des données indiquant une fonction cognitive d'une zone choisie du cerveau.

15. Dispositif de spectrophotométrie cognitive pour l'examen d'un tissu humain d'un patient par mesure des modifications du rayonnement électromagnétique diffusé ou absorbé sur une migration tissulaire, ce dispositif comprenant : une première et une seconde borne d'entrée pour introduire le rayonnement électromagnétique dans le tissu humain et au moins une source lumineuse couplée de manière optique aux bornes d'entrée pour fournir ce rayonnement électromagnétique ; une première et une seconde borne de détection et au moins un détecteur couplé à ces bornes pour détecter le rayonnement électromagnétique reçu par celles-ci ; et un dispositif de traitement comprenant un ensemble de circuits électroniques, couplés à ce détecteur pour recevoir les signaux en provenant ; ce dispositif étant caractérisé en ce que la première et la seconde borne d'entrée sont chacune adaptées pour introduire dans le tissu humain un rayonnement électromagnétique de longueur d'onde choisie, ce rayonnement provenant de cette (ces) source(s) lumineuse(s) ; en ce que cette première et cette seconde borne d'entrée sont conçues pour maintenir une première distance relative servant à définir un volume d'examen d'une première zone de tissu ; en ce que la seconde borne d'entrée et la seconde borne de détection sont conçues pour conserver une seconde distance relative servant à définir un volume d'examen d'une seconde zone de tissu quand ces bornes sont placées sur le tissu humain ; ces première et seconde distances relatives étant sensiblement les mêmes et les première et seconde zones de tissu ayant des volumes similaires ; le(s) détecteur(s) étant suffisamment indépendant pour détecter au niveau de la première borne le rayonnement venant de la (les) source(s) lumineuse(s) et ayant migré au travers de la peau et du tissu dans la première zone de tissu de la première borne d'entrée à la première borne de détection et, au niveau de la seconde borne de détection, le rayonnement de la (des) source(s) lumineuse(s) et ayant migré au travers de la peau et du tissu dans la seconde zone de tissu de la seconde borne d'entrée à la seconde borne de détection ; et en ce que le dispositif de traitement est adapté pour traiter des signaux reçus pour fournir les premières et secondes données traitées, correspondant respectivement au rayonnement détecté au niveau des première et seconde bornes de détection, ce rayonnement étant fonction de l'absorption ou la diffusion de la peau et du tissu au travers desquels il a migré, et aussi pour corréler ces premières et ces secondes données pour fournir une indication concernant une propriété physiologique ou physiopathologique du tissu examine.

16. Dispositif selon la revendication 15, caractérisé en outre en ce qu'un seul détecteur est utilisé connecté au deux bornes de détection.

17. Dispositif selon la revendication 16, caractérisé en outre en ce que le détecteur est un détecteur différentiel adapté pour comparer le rayonnement électromagnétique détecté au niveau des première et seconde bornes de détection pour générer des données traitées correspondant à un signal différente, ce signal différentiel fournissant une indication concernant cette propriété.

18. Dispositif selon la revendication 15, caractérisé de plus en ce que le dispositif de traitement est en outre adapté pour comparer les signaux du rayonnement détecté au niveau des première et seconde bornes de détection et générer des données différentielles fournissant une indication concernant cette propriété.

19. Dispositif selon la revendication 15, caractérisé en outre en ce que deux détecteurs sont utilisés, chacun étant connecté de manière optique à une borne de détection.

20. Dispositif selon la revendication 15, caractérisé en outre en ce que deux sources lumineuses sont utilisées, chacune étant connectée à une borne d'entrée respective.

21. Dispositif selon la revendication 15, caractérisé en outre en ce qu'une seule source lumineuse est connectée aux deux bornes d'entrée.

22. Dispositif selon l'une quelconque des revendications 15 à 21, caractérisé en outre en ce que les bornes d'entrée et de détection sont adaptées pour être disposées respectivement sur des zones symétriques du sein droit de du sein gauche, du bras droit et du bras gauche ou de la jambe droite et de la jambe gauche.

23. Dispositif selon l'une quelconque des revendications 15 à 21, caractérisé en outre en ce que les bornes d'entrée et de détection sont adaptées pour être disposées sur l'un des emplacements suivants : l'os frontal, l'os pariétal, l'os temporal, l'os occipital du crâne ; la distance séparant ces bornes étant maintenue à une valeur prédéterminée afin de localiser la migration du rayonnement vers la zone donnée du crâne.

24. Dispositif selon l'une quelconque des revendications 15 à 23, caractérisé en outre en ce que le dispositif de traitement est adapté pour traiter les signaux reçus par transformation de Fourrier.

25. Dispositif selon l'une quelconque des revendications 12 à 24, caractérisé en outre en ce que la (chaque) source lumineuse comprend une lampe à incandescence ou une diode électroluminescente.

26. Dispositif selon l'une quelconque des revendications 12 à 25, caractérisé en outre en ce que le (chaque) détecteur comprend une diode au silicium avec un filtre interférentiel adapté pour détecter le rayonnement de longueur d'onde donnée.

27. Dispositif selon les revendications 15 à 25, caractérisé en outre en ce que le (chaque) détecteur est un convertisseur de lumière en fréquence doté d'un filtre interférentiel adapté pour détecter le rayonnement de longueur d'onde donnée.

28. Dispositif selon la revendication 19 et la revendication 27, caractérisé en outre en ce que le dispositif de traitement comprend (a) un compteur différentiel adapté pour enregistrer les signaux différentiels reçus des convertisseurs de lumière en fréquence ; (b) un moyen de cadencement adapté pour acheminer les signaux du rayonnement détecté des convertisseurs de lumière en fréquence vers le compteur différentiel ; et (c) un convertisseur de fréquence en tension adapté pour convertir les signaux provenant du compteur différentiel.

29. Dispositif selon les deux revendications 20 et 24, caractérisé de plus en ce que le dispositif de traitement comprend en outre un transformateur de Fourrier adapté pour traiter les signaux différentiels provenant du convertisseur de fréquence en tension.

30. Dispositif selon l'une quelconque des revendications 12 à 29, caractérisé en ce qu'il comprend en outre des première et seconde barrières construites et conçues pour intercepter la lumière superficielle migrant latéralement dans la peau et la chair des bornes d'entrée vers les bornes de détection.

31. Dispositif selon l'une quelconque des revendications 15 à 21, caractérisé en outre en ce que lesdites bornes d'entrée et de détection sont adaptées pour être disposées sur des zones symétriques du sein gauche et du sein droit pour localiser la migration photonique dans deux zones correspondantes de tissu du sein ; et en ce que le dispositif de traitement est adapté pour corréler les premières et secondes données pour fournir une indication sur la tumeur présente dans une des zones du sein.

32. Dispositif selon l'une quelconque des revendications 15 à 21, caractérisé en outre en ce que lesdites bornes d'entrée et de détection sont adaptées pour être disposées sur des zones symétriques sur les côtés gauche et droit de la tête pour localiser la migration photonique dans deux zones correspondantes du cerveau ; et en ce que le dispositif de traitement est adapté pour corréler les premières et secondes données pour fournir une indication sur une tumeur présente dans une de ces zones.

33. Dispositif selon l'une quelconque des revendications 15 à 21, dans lequel les bornes d'entrée et de détection sont adaptées pour être disposées sur des zones symétriques sur les côtés gauche et droit du corps pour localiser la migration photonique dans deux zones correspondantes sur les côtés gauche et droit de ce corps ; et en ce que le dispositif de traitement est adapté pour corréler les premières et secondes données indiquant une hémorragie dans une de ces zones.
